(19) Europäisches Patentamt
European Patent Office
Office européen des brevets

(11) Numéro de publication: **0 192 554 B1**

(12) **FASCICULE DE BREVET EUROPEEN**

(45) Date de publication de fascicule du brevet: **02.01.92**

(51) Int. Cl.[5]: **C07K 5/00**, A61K 37/64

(21) Numéro de dépôt: **86400271.2**

(22) Date de dépôt: **10.02.86**

Le dossier contient des informations techniques présentées postérieurement au dépôt de la demande et ne figurant pas dans le présent fascicule.

# (54) Dérivés peptidiques inhibiteurs de la rénine et des protéases acides.

(30) Priorité: **12.02.85 FR 8501981**
**12.02.85 FR 8501982**

(43) Date de publication de la demande:
**27.08.86 Bulletin 86/35**

(45) Mention de la délivrance du brevet:
**02.01.92 Bulletin 92/01**

(84) Etats contractants désignés:
**AT BE CH DE FR GB IT LI LU NL SE**

(56) Documents cités:
**EP-A- 0 081 783**
**FR-A- 2 531 951**

(73) Titulaire: **SANOFI**
**40, Avenue George V**
**F-75008 Paris(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Wagnon, Jean Le Hameau de la Rauze**
**Villa 34 Avenue du Pont Trinquat**
**F-34000 Montpellier(FR)**
Inventeur: **Callet, Georges**
**Cité Gleurie-Bât. 2 Avenue de Maurin**
**F-34000 Montpellier(FR)**
Inventeur: **Gagnol, Jean Pierre**
**Place de L'Eglise**
**34380 Saint Martin de Londres(FR)**
Inventeur: **Nisato, Dino**
**4, Impasse de l'Olivette**
**F-34680 Saint Georges d'Orques(FR)**
Inventeur: **Cazaubon, Catherine**
**9, rue Pierre Boissier**
**F-34000 Montpellier(FR)**

(74) Mandataire: **Gillard, Marie-Louise et al**
**Cabinet Beau de Loménie 55, Rue d'Amsterdam**
**F-75008 Paris(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

EP 0 192 554 B1

## Description

La présente invention concerne des nouveaux dérivés peptidiques inhibiteurs de la rénine et des protéases acides. Elle concerne également un procédé pour leur obtention et leur application en thérapeutique.

En 1970, UMEZAWA a isolé, à partir d'une culture de streptomyces, un pentapeptide désigné sous le nom de Pepstatine dont la structure a été établie ultérieurement et répond à la formule : Isovaléryl - L-valyl - L-valyl - Statyl - L-alanyl - Statine, dans laquelle on désigne sous le nom de "statine" un acide aminé nom usuel, l'acide (3S, 4S) amino-4 hydroxy-3 méthyl-6 heptanoïque.

Il a été montré que la pepstatine est un inhibiteur de protéases acides et agit en particulier sur la pepsine, la cathepstine D et la résine. Spécialement, la rénine, enzyme d'origine rénale, intervient dans la séquence angiotensinogène, angiotensine I, angiotensine II au niveau de la transformation de l'angiotensinogène en engiotensine.

L'engiotensine II, étant un puissant agent vasoconstricteur, intervient dans la régulation de la pression artérielle. On a envisagé d'utiliser la pepstatine pour lutter contre l'hypertension artérielle chez l'homme. Toutefois, la pepstatine agissant sur l'ensemble des protéases acides et présentant une faible solubilité en milieux aqueux et une faible affinité pour la rénine, son emploi en thérapeutique s'est avéré difficile. Des dérivés de la pepstatine ont été décrits dans la littérature scientifique. Par exemple, on a tenté de solubiliser la pepstatine par allongement de la chaîne peptidique (J. Cardiovasc. Pharmacol., 1980, 2, 687-698).

On a également décrit dans FR-A-2 531 951 des dérivés peptidiques inhibiteurs de protéases acides qui répondent à la formule générale :

R-X-Y-Statyl-Ala-Statyl-OR'

dans laquelleX et Y, identiques ou différents, désignent des acides aminés de configuration L ou D, choisis parmi la valine, la leucine, l'isoleucine, la phénylalanine, la tyrosine, le tryptophane, l'histidine et la proline, à la condition que X et Y ne soient pas simultanément la valine.

La présente invention concerne au contraire des peptides dont le modèle est construit sur celui de la pepstatine. Dans certains cas, l'introduction d'acides aminés non naturels permet d'augmenter la solubilité de ces composés et leur résistance à la protéolyse. De manière tout à fait surprenante, on a trouvé que les peptides selon l'invention, qui portent des résidus hydrophiles sur différents sites, présentent un niveau d'activité élevé et très supérieur à celui de la pepstatine en tant qu'inhibiteur de la rénine humaine.

La présente invention a pour objet des peptides présentant un niveau élevé d'activité en tant qu'inhibiteurs de la rénine et d'autres protéases acides.

Dans la présente description et dans les revendications, les abréviations suivantes seront utilisées :

Acides aminés et groupes protecteurs ou activateurs.

Ces abréviations sont en accord avec celles indiquées par la Commission de Nomenclature de l'IUPAC-IUB section Biochimie. Les recommandations les plus récentes sont rapportées dans Eur. J. Biochem., 1984, 138, 5-7 et 9-37.

Acides aminés et dérivés :

Ala : Alanine
Asn : Asparagine
Asp : Acide aspartique
Gln : Glutamine
Gly : Glycine
His : Histidine
Ile : Isoleucine
Leu : Leucine
Met : Methionine
Nle : Norleucine
Nva : Norvaline
Phe : Phénylalanine
Ser : Sérine
Sta : Statine
AHPPA : Acide amino-4 hydroxy-3 phényl-5 pentanoïque
ACHPA : Acide amino-4 cyclohexyl-5 hydroxy-3 pentanoïque

$Met(O_2)$ : Méthionine dioxyde
$(PhCH_2)Asp$ : Acide O.benzyl aspartique
Abu : Acide amino-2 butyrique
(Pyr-2)Ala : (Pyridyl-2) Alanine

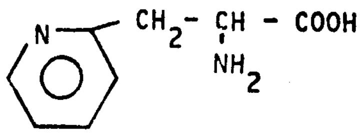

(Pyr-3)Ala : (Pyridyl-3) Alanine
(Pyr-4)Ala : (Pyridyl-4) Alanine
(tauMe)His : (télé-méthyl) Histidine

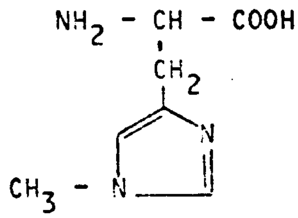

(tauEt)His : (télé-éthyl) Histidine
(piMe)His : (pros-méthyl) Histidine

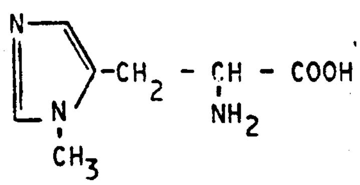

[(méthyl-2)thiazolyl-4]Gly :

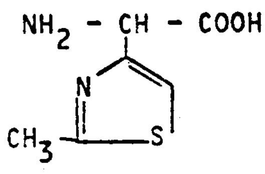

[(méthyl-1) imidazolyl-2] Ala :

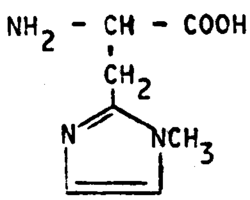

Hph : Homophénylalanine
Phg : Phénylglycine
Cpg : Cyclopentylglycine
$(CH_3CH_2O)Ser$ : Ether éthylique de sérine
$((CH_3)_3CO)Ser$ : Ether t.butylique de sérine

$(PhCH_2O)Ser$ : Ether benzylique de sérine
$((Pyr\text{-}2)CH_2O)Ser$ : Ether (pyridyl-2) méthylique de sérine
(Pentyl)Ala : Acide amino-2 octanoïque
(Ph)Gln : N(phényl)glutamine
(Ph)Asn : N(phényl)asparagine

Ces acides aminés sont, sauf indication contraire, de configuration L. Sta, AHPPA, ACHPA sont, sauf indication contraire, de configuration 3S, 4S.

<u>Groupes protecteurs et activateurs</u> :

Ac : Acétyle
Boc : t.butyloxycarbonyle
$(Boc)_2O$ : Anhydride bis terbutyloxycarbonique
HONSu : N-hydroxysuccinimide
OEt : Ester éthylique
OMe : Ester méthylique
ONp : Ester p-nitrophénylique
ONSu : Ester de N-hydroxysuccinimide
OTcp : Ester trichloro-2,4,5 phénylique
iVa : Isovaléryle
Z : Benzoyloxycarbonyle

De plus, on utilisera les abréviations suivantes :

AcOEt : Acétate d'éthyle
AcOH : Acide acétique
Bop : Hexafluorophosphate de benzyloxy tris-diméthylamino phosphonium
CCM : Chromatographie en couche mince
DCCI : Dicyclohexylcarbodiimide
DCHA : Dicyclohexylamine
DCU : Dicyclohexylurée
DIPEA : Diisopropyléthylamine
DMF : Diméthylformamide
DMSO : Diméthylsulfoxyde
Ether : Ether éthylique
HOBt : Hydroxy-1 benzotriazole
$KHSO_4\text{-}K_2SO_4$ : Solution aqueuse contenant 16,6 g de bisulfate de potassium et 33,3 g de sulfate de potassium pour 1 l
MeOH : Méthanol
NEM : N-éthyl morpholine
NMM : N-méthyl morpholine
TA : Température ambiante
TFA : Acide trifluoracétique
min : minutes
h : heures.

Les composés selon l'invention répondent à la formule générale suivante :

```
R1-NH-CH-C-NH-CH-C-NH-CH-CHOH-CH2-C-X-Y-R4
      |  ||     |  ||   |            ||
      R2 O      R3 O    CH2          O
                        |
                        Z1                      (I)
```

dans laquelle :

$R_1$ représente un groupe acyle choisi parmi les groupes alkylcarbonyle, alcoxycarbonyle, arylcarbonyle, arylalkylcarbonyle, arylalcoxycarbonyle, hétérocyclylcarbonyle, hétérocyclylalkylcarbonyle dans lequel le groupe alkyle est éventuellement substitué par un groupe hydroxy, hétérocyclylcarbonylalkylcarbonyle,

hétérocyclylalcénylcarbonyle, cycloalkylcarbonyle, ou un groupe (alkyle contenant jusqu'à 6 atomes de carbone)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle contenant jusqu'à 6 atomes de carbone ;

$R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle ;

$R_3$ représente un alcényle contenant jusqu'à 6 atomes de carbone, un phényle, un naphtyle, un cycloalkyle contenant 3 à 6 atomes de carbone, un groupe hétérocyclique monocyclique à 5 ou 6 chaînons non substitué ou substitué par un alkyle contenant jusqu'à 6 atomes de carbone ou trifluorométhyle choisi parmi la pyrolidine, l'inidazole, le thiazole, le thiophère, le furanne, le pyrole, le triazole, l'oxazole, l'isoxazole, la pyridine, les thiaziazoles; ou bien un alkyle contenant jusqu'à 6 atomes de carbone substitué par un groupe di(alkyle contenant jusqu'à 6 atomes de carbone)-amino, par un carboxyle estérifié par un alkyle contenant jusqu'à 6 atomes de carbone ou un benzyle, par un carbamoyle libre ou substitué par un ou deux alkyles contenant jusqu'à 6 atomes de carbone ou par un phényle, par un groupe alcoxy contenant jusqu'à 6 atomes de carbone ou benzyloxy, par un groupe pyridylméthyloxy, par un groupe (alkyle contenant jusqu'à 6 atomes de carbone) sulfinyle ou (alkyle contenant jusqu'à 6 atomes de carbone)sulfonyle, par un cycloalkyle contenant de 3 à 6 atomes de carbone ou par un groupe hétérocyclique monocyclique à 5 ou 6 chaînons non substitué ou substitué avec un alkyle contenant jusqu'à 6 atomes de carbone ou un trifluorométhyle, à la condition que $R_3$ ne soit pas le groupe (imidazolyl-4)-méthyle;

$R_4$ représente un hydroxyle, un alcoxy contenant jusqu'à 6 atomes de carbone, un benzyloxy ou un groupe amino libre ou substitué par 1 ou 2 alkyles contenant jusqu'à 6 atomes de carbone;

Z1 représente l'isopropyle, le phényle ou le cyclohexyle formant respectivement avec le radical

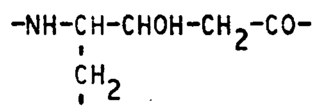

le résidu de l'aminoacide statine, à savoir, l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA) ;

X-Y est un dipeptide choisi parmi Ala-Sta, Ala-Leu, Leu-Phe, Val-Sta, Abu-Sta, Ile-Ser, Phg-Sta.

La présente invention comprend également les sels éventuels pharmaceutiquement acceptables des peptides de formule (I) avec des acides minéraux ou organiques ou des métaux alcalins ou alcalino-terreux.

Le terme "alkyle" désigne les radicaux d'hydrocarbures aliphatiques saturés ou insaturés contenant 1 à 10 atomes de carbone. Les groupes "alkyle" préférés aux fins de l'invention sont les groupes alkyle inférieur tels que définis ci-après.

Les expressions "alkyle inférieur", "alcényle inférieur" et "alkylidène inférieur", telles qu'utilisées ici, désignent les radicaux d'hydrocarbures aliphatiques saturés ou insaturés contenant jusqu'à 6 atomes de carbone.

L'expression "alcoxy inférieur" représente le groupe hydroxyle substitué par un groupe alkyle inférieur tel que défini ci-dessus.

L'expression "hétérocycle monocyclique à 5 ou 6 chaînons" inclut la pyrrolidine, l'imidazole, le thiazole, le thiophène, le furanne, le pyrrole, le triazole, l'oxazole, l'isoxazole, la pyridine, les thiadiazoles.

Par "groupe protecteur", on entend un groupe protecteur utilisé normalement dans la chimie des peptides, par exemple Boc, Z ou iVa.

L'expression "groupe acyle" utilisée pour la définition de $R_1$ inclut les résidus d'acides carboxyliques aliphatiques, alicycliques, aromatiques ou hétérocycliques. Des groupes acyles préférés sont le résidu de l'acide carbonique estérifié, notamment les groupes Boc et Z, le résidu d'acides alconoïques contenant de 2 à 6 atomes de carbone, notamment le groupe iVa, le résidu de l'acide cyclohexylcarboxylique, le résidu d'acides phénylaliphatiques, notamment les groupes phénylacétyle, 3-phénylpropionyle, dibenzylacétyle, le résidu d'acides arylcarboxyliques, tels que l'acide naphtoïque, biphénylcarboxylique et l'acide benzoïque non substitué ou substitué sur le noyau phényle, notamment le groupe benzoyle, le résidu d'un acide carboxylique dont le carboxyle est lié à un hétérocycle monocyclique à 5 ou 6 chaînons, notamment les groupes pipéridinylcarboxyliques, les groupes picolinoyle, nicotinoyle et isonicotinoyle et le résidu d'acides alcanoïques ou alcénoïques, tels que l'acide acétique, l'acide propionique, l'acide butyrique, l'acide

valérique et leurs dérivés oméga-hydroxy ou oméga-oxo, substitués en position oméga par un hétérocycle monocyclique à 5 ou 6 chaînons comme exemplifiés ci-dessus.

Plus particulièrement, la présente invention concerne, d'une façon préférentielle, les dérivés peptidiques de formule (I) dans laquelle $R_2$, $R_3$, $R_4$, X, Y et $Z_1$ sont tels que définis ci-dessus et $R_1$ représente l'un des groupements suivants :

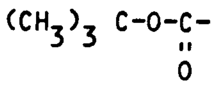

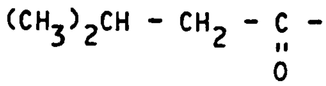

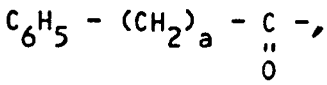
où a = 0,1 ou 2

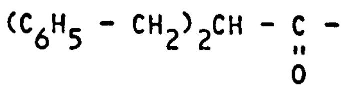

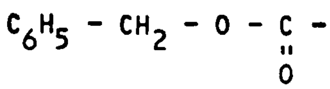

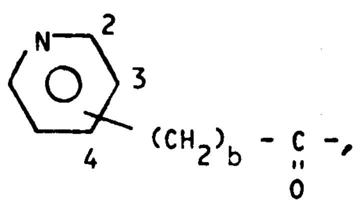
où b = 0,1,2,3,4,5 ou 6

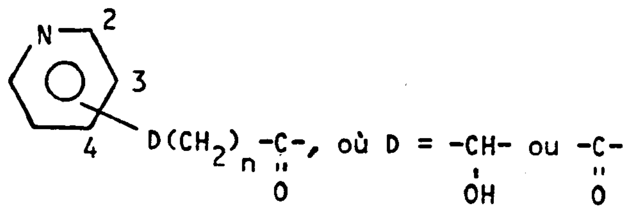

et n = 1,2,3,4 ou 5

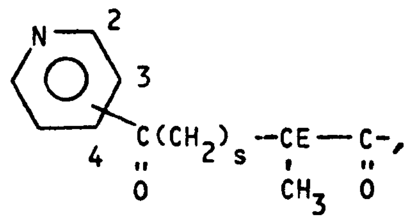

où E = -H ou $-CH_3$ et s = 1,2,3 ou 4

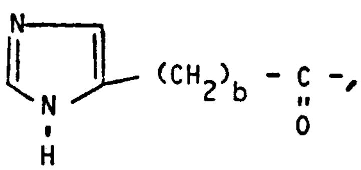

où b = 0,1,2,3,4,5 ou 6

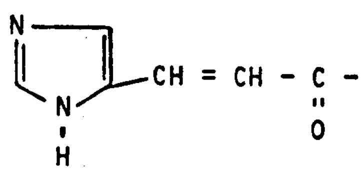

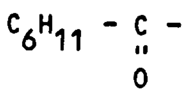

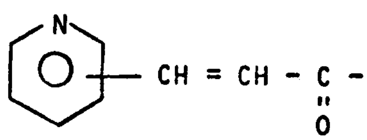

$NH_2CH_2CH_2$ - $SO_2$ -
$BocNHCH_2CH_2$ - $SO_2$ -
Z-$NHCH_2CH_2$ - $SO_2$ -
A-$SO_2$ - où A est un alkyle inférieur
$C_6H_5$ - $(CH_2)a$ - $SO_2$ -, où a = 0,1 ou 2,

Particulièrement préférés sont les dérivés peptidiques de formule (I) dans laquelle $R_2$, $R_3$, $R_4$, X, Y et $Z_1$ sont tels que définis ci-dessus et $R_1$ représente un groupe acyle choisi parmi :

- isolvaléryle,
- (pyridyl-2)-4 oxo-4 butyryle,
- (pyridyl-2)-4 hydroxy-4 butyryle,
- (pyridyl-3)-3 propionyle,
- (pyridyl-3)-4 butyryle,
- nicotinoyle,

- benzène sulfonyle.

De plus, sont particulièrement préférés les composés de formule (I) dans laquelle $R_1$, $R_2$, $R_4$, X, Y et $Z_1$ sont tels que définis précédemment et $R_3$ est tel que le résidu -NH-CH($R_3$)-CO- représente le résidu (tauMe)His.

Les produits selon l'invention peuvent être préparés selon les méthodes habituelles de la chimie des peptides. Plus particulièrement, à partir d'un composé de formule :

H-Y-$R'_4$

dans laquelle $R'_4$ représente un alcoxy inférieur, un benzyloxy ou un groupe amino libre ou substitué par 1 ou 2 alkyles inférieurs et Y est choisi parmi les résidus des acides aminés Sta, Leu, Phe et Ser, on couple, étape par étape, les divers acides aminés convenablement protégés, le produit obtenu à chaque étape étant déprotégé, selon des procédés connus, avant d'être soumis à un nouveau couplage, chacune des opérations de couplage étant effectuée en utilisant, soit un ester activé de l'aminoacide à coupler, soit l'aminoacide N-protégé en présence de dicyclohexylcarbodiimide. Le produit de départ est avantageusement un ester d'alkyle inférieur de l'acide aminé C terminal sur lequel est condensé l'aminoacide suivant de la séquence. Après libération de la fonction amine du dipeptide, on procède à l'élongation de la chaîne peptidique par couplage de l'acide aminé suivant, convenablement protégé. Chaque phase de couplage est suivie d'un opération sélective de libération de l'amine qui va entrer en réaction lors de la création de la liaison peptidique suivante. Les différentes opérations de couplage sont effectuées soit en utilisant un ester activé de l'aminoacide à coupler, soit en utilisant l'aminoacide N-protégé en présence de dicyclohexylcarbodiimide. Les phases de déprotection sélective de l'amine sont effectuées selon la nature du groupe protecteur utilisé soit par hydrogénolyse, soit par acidolyse en milieu acide fort tel que l'acide trifluoracétique. Lorsque l'acide aminé à introduire dans la séquence possède dans sa chaîne latérale une fonction susceptible de réagir, il convient de bloquer celle-ci par un groupe protecteur convenable que l'on élimine ultérieurement.

La protection de l'aminoacide initial par le groupe $R_1$ s'effectue selon des méthodes connues, avant le couplage du résidu :

$R_1$ - NH - $CHR_2$ - CO -

avec l'aminoacide suivant.

Le résidu - NH - $CHR_3$ - CO - est un aminoacide non naturel, préparé selon des méthodes connues; il est ensuite couplé avec l'aminoacide suivant selon la méthode habituelle.

Les peptides (I) sous forme acide ($R_4$ = OH) peuvent être obtenus à partir des esters correspondants par saponification en milieu alcalin dilué. On peut également préparer leurs sels. Les peptides (I) sous forme amide ($R_4$ = $NH_2$ ou $R_4$ = $N(Alk)_2$) sont obtenus directement en prenant comme produit de départ les aminoamides qui existent commercialement.

Selon le schéma général, il est possible soit de préparer l'ensemble de la séquence désirée, soit de préparer des fragments de cette séquence que l'on couple finalement pour obtenir le peptide désiré.

Les sels éventuels pharmaceutiquement acceptables des peptides selon l'invention avec des acides minéraux ou organiques ou des métaux alcalins ou alcalino-terreux sont formés par des méthodes classiques.

Les composés de la présente invention ont une action inhibitrice sur l'activité rénine plasmatique humaine très importante et de façon générale nettement supérieure à celle du produit naturel : la pepstatine ; à ce titre, ils peuvent être utilisés dans le traitement de l'hypertension artérielle.

Ils possèdent également une action inhibitrice marquée sur les protéases acides, notamment la pepsine. On peut donc envisager l'utilisation des produits selon l'invention dans les domaines thérapeutiques où l'inhibition de tels systèmes enzymatiques est justifiée, notamment, outre l'hypertension artérielle, l'ulcère gastroduodénal et les affections inflammatoires.

La présente invention a aussi pour objet les compositions pharmaceutiques convenant comme antihypertenseurs et contenant les peptides de formule (I) ou leurs sels pharmaceutiquement acceptables en tant que principe actif.

Les peptides de la présente invention peuvent être utilisés en thérapeutique par voie injectable : intraveineuse, intramusculaire ou sous-cutanée. Ils sont utilisés dans un solvant, tel que le sérum physiologique (solution saline isotonique) ou dans un tampon, tel que le tampon phosphate ; ils peuvent également être mis en suspension dans un agent diluant, aqueux ou non aqueux, pharmaceutiquement acceptable. Chaque unité de dosage peut contenir de 1 à 1000 mg de principe actif.

La quantité de principe actif à utiliser varie suivant les effets thérapeutiques recherchés, la gravité de l'affection à traiter et la voie d'administration choisie. Elle doit être déterminée pour chaque patient et est le plus souvent comprise entre 0,100 g et 2 g de principe actif.

Les exemples suivants, non limitatifs, sont donnés à titre d'illustration de la présente invention.

Dans tous ces exemples, le pH des solutions dans un solvant organique est mesuré ou contrôlé en utilisant du papier pH indicateur humide.

Les points de fusion indiqués (Fc) sont mesurés au tube capillaire.

Enfin, les spectres de résonance magnétique nucléaire ont été enregistrés à 250 MHz en solution dans le DMSO, l'étalon interne étant l'hexaméthyldisiloxanne.

Les abréviations suivantes sont utilisées :

s : singulet
d : doublet
m : multiplet ou massif
q : quadruplet.

De plus,

. H ar signifie H aromatique
. H im signifie H imidazolique
. H pyr signifie H de la pyridine.

Les déplacements chimiques (delta) sont mesurés en ppm.

Exemple 1

Boc-Phe-($PhCH_2$)Asp-Sta-Ala-Leu-OMe (SR 42926).

1. Boc-Ala-Leu-OMe.

Dans 50 ml de $CH_2Cl_2$, on dissout successivement à TA 2,86 g de Boc-Ala-ONSu, 1,81 g de Leu-OMe, HCl et 1,15 g de NEM. On vérifie que le pH est à 6-7, sinon, on l'ajuste par adjonction de NEM. On agite 4 h à TA, puis lave successivement la solution organique par $KHSO_4$-$K_2SO_4$ à 5 %, de l'eau, $NaHCO_3$ à 5 % ; on sèche sur $Na_2SO_4$ puis évapore le solvant et on obtient une huile.

Rendement : 2,93 g (92 %).

2. Ala-Leu-OMe, TFA.

360 mg du produit précédent sont recouverts de 5 ml de TFA. Après 20 min, le TFA est évaporé, on reprend le résidu par un mélange, à volume égal, pentane-éther.Par grattage, un solide blanc apparaît, il est essoré, rincé à l'éther, séché.

Rendement : 320 mg (85 %).

3. Boc-Sta-Ala-Leu-OMe.

330 mg du sel de TFA précédemment obtenu sont solubilisés dans 30 ml de dioxanne contenant 230 mg de NEM, on ajoute 275 mg de Boc-Sta-OH, 206 mg de DCCI et 135 mg de HOBt, le pH est ajusté à une valeur de 6-7 par NEM si nécessaire, puis on agite 24 h à TA. On filtre la DCU, évapore le solvant, dissout le résidu dans un mélange, à volume égal, AcOEt-hexane et chromatographie sur une colonne de gel de silice 60 Merck dans le même mélange de solvant en éluant par 250 ml du même mélange de solvant, puis 250 ml du mélange dans la proportion 75/25 en volume, puis 100 ml d'AcOEt. Les fractions contenant le produit sont évaporées, reprises dans l'éther, essorées, séchées.

Rendement : 380 mg (80 %).

4. Boc-($PhCH_2$)Asp-Sta-Ala-Leu-OMe.

On traite à 0°C pendant 15 min 473 mg du peptide précédemment obtenu par 8 ml de TFA ; on concentre sous vide à t = 25°C, reprend par de l'éther et évapore. On obtient un solide que l'on reprend par $CH_2Cl_2$, on neutralise à froid le sel par un léger excès de NMM, puis on ajoute 405 mg de Boc-($PhCH_2$)Asp 191 mg de HOBt, 258 mg de DCCI. Le pH est ajusté à 7 par addition de NMM puis on agite 18 h. On lave ensuite par une solution de $KHSO_4$ (environ 1M), par une solution de $NaHCO_3$ (environ 1M), sèche et concentre. Après chromatographie sur colonne de silice avec comme éluant AcOEt, on obtient 169

mg du produit attendu qui cristallise à l'évaporation ;
Fc = 147-149° C.

5. SR 42926.

On traite 169 mg du produit précédent par 8 ml de TFA à 0° C pendant 15 min. On concentre sous vide à 25° C, reprend par l'éther et évapore. On obtient une huile que l'on reprend par $CH_2Cl_2$, on neutralise à froid le sel par un léger excès de NMM. Un mélange de 100 mg de Boc-Phe-ONp, 38 mg de HOBt, 26 mg de NMM est agité 2 h dans du $CH_2Cl_2$ puis ajouté au sel précédent. Après 24 h d'agitation, on lave la phase organique avec une solution de $KHSO_4$, de $NaHCO_3$, sèche et concentre. Après chromatographie sur silice (éluant AcOEt), on obtient le produit attendu qui cristallise à l'évaporation du solvant.

Rendement : 103 mg (50 %) ; Fc = 154-155° C.

SPECTRE DE RMN

| Delta | Aspect | Protons | Attribution |
|---|---|---|---|
| 8,34-7,05 | m | 14 H | 4 NHCO<br>10 H ar |
| 6,86 | d, J=8 Hz | 1 H | $NHCO_2$ |
| 5,04 | s | 2 H | $CO_2CH_2C_6H_5$ |
| 3,55 | s | 3 H | $OCH_3$ |
| 2,20-2,00 | m | 2 H | $CH_2CO$ (Sta) |
| 1,20 | s | 9 H | $C(CH_3)_3$ |
| 1,13 | d, J=7 Hz | 3 H | $CH_3$ (Ala) |
| 0,86-0,69 | m | 12 H | $(CH_3)_2CH$ |

Exemple 2

Boc-Phe-((méthyl-2) thiazolyl-4)Gly-Sta-Ala-Leu-OMe (SR 43059).

1. (R,S) ((méthyl-2) thiazolyl-4) glycinate d'éthyle.

Ce produit est préparé selon la méthode décrite dans J. Med. Chem, 1973, 16, 978-984.

2. (R,S) ((méthyl-2) thiazolyl-4) N-tertiobutyloxycarbonyl glycinate d'éthyle.

6,5 g du composé préparé précédemment sont agités 3 jours dans 60 ml d'eau avec 8,1 g de $Boc_2O$, 32,5 ml de dioxanne et de la triéthylamine pour obtenir un pH alcalin. On concentre ensuite à 30° C, reprend à l'éther, lave à l'eau puis avec une solution de $KHSO_4$, à l'eau, une solution de $NaHCO_3$, sèche et concentre. On obtient 8,5 g d'une huile jaune.

3. (R,S) ((méthyl-2) thiazolyl-4) N-tertiobutyloxycarbonyl glycine.

4,5 g du composé précédemment obtenu sont mis à réagir dans 80 ml de dioxanne avec 0,72 mg de soude dissous dans 15 ml d'eau. On suit la réaction par CCM. Après 2 h, on rajoute 0,6 g de soude dans 2

ml d'eau. Au bout de 3 h 45 min, il n'y a plus de produit de départ. On ramène à pH 5 par 15 ml d'HCl N, concentre à 30 °C, reprend à l'eau, acidifie par $KHSO_4$, extrait par AcOEt, lave à l'eau, sèche et concentre. On recueille 2,7 g de poudre jaune.

4. Boc-Sta-Ala-Leu-OMe.

Ce peptide est préparé selon la méthode décrite à l'exemple 1.

5. (R,S) Boc-((méthy-2)thiazolyl-4)Gly-Sta-Ala-Leu-OMe.

473 mg du produit précédent sont placés dans 5 ml de TFA à 0 °C. Après 20 min, on concentre, reprend 3 fois par de l'éther anhydre puis concentre au maximum. Le produit obtenu est dissous dans 20 ml de $CH_2Cl_2$ et amené vers pH 7 par addition de DIPEA. On ajoute ensuite 260 mg du composé obtenu à l'étape 3 et 441 mg de Bop. Après 4 jours d'agitation à pH 7, on traite à l'eau salée puis à l'eau bicarbonatée et à l'eau, on sèche et concentre, puis on effectue une chromatographie sur silice. Par élution avec AcOEt puis un mélange AcOEt-MeOH à 9/1 en volume, on recueille une fraction solide amorphe de 607 mg par précipitation dans le pentane.

6. SR 43059.

251 mg du composé obtenu précédemment sont placés dans 4 ml de TFA à 0 °C puis subissent le traitement habituel. Le sel de TFA obtenu est placé dans 15 ml de $CH_2Cl_2$, le milieu réactionnel est amené à pH 7 par addition de DIPEA puis on ajoute 149 mg de Boc-Phe-ONp et 61,2 mg d'HOBt. Après 24 h, on lave de la même façon qu'à l'étape précédente puis on chromatographie sur silice en éluant par un mélange AcOEt-AcOH à 9/1 en volume. On recueille 230 mg de poudre blanche.

SPECTRE DE RMN

| Delta | Aspect | Protons | Attribution |
|---|---|---|---|
| 7 - 8,4 | m | 11 H | 5 NH<br>5 H ar<br>1 H thiazoliques |
| 3,5 | s | 3 H | $OCH_3$ |
| 1,25 | s | 9 H | $C(CH_3)_3$ |

Exemple 3

Boc-Phe-(Pyr-3)Ala-Sta-Leu-Phe-OMe (SR 43064 et SR 43066).

1. (R,S) Acétamido-2 éthoxycarbonyl-2 (pyridyl-3)-3 propionate d'éthyle.

A 11 g d'une solution de sodium dans 300 ml de EtOH, on ajoute 48 g d'acétamidomalonate de diéthyle puis, en portant à reflux, on additionne 36 g de chlorhydrate de chlorométhyl-3 pyridine, on conserve 5 h à reflux. On évapore EtOH, reprend par l'eau, extrait, sèche, concentre et chromatographie sur silice. Le mélange $CH_2Cl_2$-AcOEt, à 60/40 en volume, élue 23 g du composé attendu.

2. (R,S) (Pyr-3)Ala, 2 HCl.

15 g du diester précédent sont portés à reflux dans 200 ml d'HCl 5 N pendant 5 h. On concentre à sec, ajoute EtOH, évapore. Le procédé est réitéré jusqu'à recristallisation du dichlorhydrate : 15 g.

3. (R,S) Boc(Pyr-3)Ala.

1 g du dichlorhydrate précédent est dissous dans un mélange à volume égal eau-dioxanne. On ajoute 350 mg de soude et 1 g de $(Boc)_2O$ puis on agite une nuit à TA. Le dioxanne est évaporé, on ajoute de l'eau, neutralise par 8 g d'Amberlite CG 120 I (forme acide), agite 1 h à TA puis le milieu est mis sur une colonne contenant 8 g de la même résine. On élue par de l'eau distillée jusqu'à obtenir pH 5 puis élue par une solution d'ammoniaque à 4 % dans l'eau distillée. Les eaux sont concentrées. On recueille 0,69 g du produit attendu.

4. Boc-(Pyr-3)Ala-Sta-OMe.

On agite pendant 3 jours à TA dans 10 ml d'acétonitrile un mélange de 465 mg de Sta-OMe, TFA, 500 mg de Boc-(Pyr-3)-Ala en présence de 780 mg de Bop, le milieu étant maintenu vers pH 7 par addition de DIPEA. On évapore alors à siccité, ajoute de l'eau, extrait par AcOEt, lave par une solution de $Na_2CO_3$, de l'eau, de l'eau saline, puis on sèche sur $MgSO_4$ et évapore à siccité. Le résidu est chormatographié sur gel de silice par un mélange éluant $CH_2Cl_2$-AcOEt à 1/9 en volume qui élue successivement :
- l'isomère A le moins polaire : 150 mg ;
- l'isomère B le plus polaire : 180 mg.

5. Leu-Phe-OMe.

Ce dipeptide est préparé selon le procédé de couplage décrit dans les exemples précédents.

6. Boc-(Pyr-3)Ala-Sta-Leu-Phe-OMe.

Ce composé est d'abord préparé à partir de l'isomère A isolé à l'étape 2. 200 mg de cet isomère A sont hydrolysés à TA pendant 1 h dans un mélange à volume égal eau-dioxanne. On évapore partiellement sous pression réduite à TA, ajoute une trace d'HCl pour amener le pH vers 4, puis évapore à siccité et sèche sous vide. Le résidu est repris dans 10 ml d'acétonitrile, on ajoute 200 mg de Leu-Phe-$OCH_3$, TFA, 230 mg de Bop et maintient pendant 18 h le milieu réactionnel vers pH 7 par addition de DIPEA. On évapore ensuite à siccité, ajoute de l'eau bicarbonatée, extrait par AcOEt. La phase organique, lavée par de l'eau, de l'eau saline puis séchée sur $MgSO_4$, livre le peptide brut après évaporation. Il est purifié par chromatographie sur gel de silice en utilisant comme éluant $CH_2Cl_2$-AcOEt à 1/4 en volume. On obtient 300 mg de produit.

7. SR 43066.

197 mg du composé précédent sont mis en contact à 0°C pendant 20 min avec 5 ml de TFA, le solvant est alors évaporé, et le sel obtenu séché sous vide. Le résidu est mis en suspension dans $CH_2Cl_2$ et neutralisé par le DIPEA. On ajoute, en solution dans $CH_2Cl_2$, 110 mg de Boc-Phe-ONp et 45 mg de HOBt ; le mélange est alors agité pendant 18 h à TA en maintenant le pH vers 7 par addition de DIPEA. On évapore à siccité, ajoute de l'eau carbonatée et extrait par AcOEt. Après lavages à l'eau et séchage sur $MgSO_4$, le résidu est chromatographié sur gel de silice (éluant AcOEt). On obtient 150 mg du SR 43066.

8. SR 43064.

A partir de l'isomère B isolé à l'étape 4, on opère selon les étapes 4 et 5 pour préparer le SR 43064.

SPECTRE DE RMN DU SR 43066

| Delta | Aspect | Protons | Attribution |
|---|---|---|---|
| 6,80 | d, J=8 Hz | 1 H | NH - Boc |
| 4,84 | d, J=4 Hz | 1 H | OH (Sta) |
| 3,50 | s | 3 H | $OCH_3$ |
| 2,16-2,05 | m | 2 H | $CH_2$-CO (Sta) |
| 1,20 | s | 9 H | $(CH_3)_3C$ |

SPECTRE DE RMN DU SR 43064

| Delta | Aspect | Protons | Attribution |
|---|---|---|---|
| 6,85 | d, J=8 Hz | 1 H | NH - Boc |
| 4,83 | d, J=4 Hz | 1 H | OH (Sta) |
| 3,50 | s | 3 H | $OCH_3$ |
| 2,10-1,95 | m | 2 H | $CH_2CO$ (Sta) |
| 1,21 | s | 9 H | $(CH_3)_3C$ |

Exemple 4

Boc-Phe-(Pyr-4)Ala-Sta-Leu-Phe-OMe (SR 43065 et SR 43144).

1. Boc-(Pyr-4)Ala-Sta-OMe.

En opérant comme dans l'exemple précédent, on prépare d'abord Boc-(Pyr-4)Ala puis on couple ce produit avec Sta-OMe, TFA. On isole successivement 2 isomères par chromatographie :

- isomère A le moins polaire ;
- isomère B.

2. SR 43065.

Ce composé est obtenu à partir de l'isomère A en opérant comme dans l'exemple précédent.

3. SR 43144.

Ce composé est obtenu à partir de l'isomère B en opérant comme dans l'exemple précédent.

SPECTRE DE RMN DU SR 43065

| Delta | Aspect | Protons | Attribution |
|---|---|---|---|
| 6,82 | d, J=8 Hz | 1 H | NH - Boc |
| 4,85 | d, J=4 Hz | 1 H | CH (Sta) |
| 3,50 | s | 3 H | $OCH_3$ |
| 2,10-2,07 | m | 2 H | $CH_2CO$ (Sta) |
| 1,21 | s | 9 H | $(CH_3)_3C$ |

SPECTRE DE RMN DU SR 43144

| Delta | Aspect | Protons | Attribution |
|---|---|---|---|
| 6,87 | d, J=8 Hz | 1 H | NH - Boc |
| 4,85 | d, J=4 Hz | 1 H | OH (Sta) |
| 3,50 | s | 3 H | $OCH_3$ |
| 2,10-2,00 | m | 2 H | $CH_2CO$ (Sta) |
| 1,23 | s | 9 H | $(CH_3)_3C$ |

Exemple 5

Boc-Phe-(Pyr-2)Ala-Sta-Leu-Phe-OMe (SR 43150 et SR 43151).

1. (R,S) Boc-Phe-(Pyr-2)Ala.

On agite pendant 48 h à TA dans 30 ml du mélange DMF-dioxanne à 1/2 en volume, 1 g de dichlorhydrate de pyridyl-2 alanine, 1,65 g de Boc-Phe-ONp en présence de 2,5 ml de triéthylamine. On filtre un léger insoluble, évapore la solution à siccité et chromatographie le résidu sur gel de silice. Le mélange $CHCl_3$-MeOH, $NH_4OH$ (84/15/5 en volume) élue le dipeptide sous forme d'une mousse soluble dans l'eau. Rendement : 58 %.

2. Boc-Phe-(Pyr-2)Ala-Sta-OMe.

A 808 mg du produit précédent à 0°C dans $CH_2Cl_2$, on ajoute 880 mg de Bop et DIPEA ; puis, après 15 min de contact, 600 mg de Sta-OMe, TFA et on amène le pH vers 6-7 par addition de DIPEA. On agite 4 jours à TA puis évapore à siccité, ajoute de l'eau carbonatée et extrait par AcOEt. Après lavage par de l'eau et de l'eau saline, on isole une huile. 850 mg sont chromatographiés sur gel de silice, en éluant par AcOEt, on isole successivement :

- le tripeptide le moins polaire A, homogène en CCM ;
- le tripeptide te plus polaire B contenant 30 % de A.

3. SR 43150.

Ce composé est obtenu à partir de l'isomère A en opérant selon la méthode décrite à l'exemple 3.

4. SR 43151.

Ce composé est obtenu à partir de l'isomère B et contient, après purification,30 à 40 % de SR 43150.

SPECTRE DE RMN DU SR 43150

| Delta | Aspect | Protons | Attribution |
|---|---|---|---|
| 6,86 | d, J=8 Hz | 1 H | NH - Boc |
| 4,76 | d, J=4 Hz | 1 H | OH (Sta) |
| 3,50 | s | 3 H | $OCH_3$ |
| 2,13-2,07 | m | 2 H | $CH_2CO$ (Sta) |
| 1,22 | s | 9 H | $(CH_3)_3C$ |

Exemple 6

Boc-Phe-Met($O_2$)-Sta-Ala-Sta-OMe (SR 42845).

1. Z-Ala-Sta-OMe.

On dissout à température ambiante,dans 35 ml de DMF, 1,44 g de H-Sta-OMe, TFA, 595 mg de NEM, 2,06 g de Z-Ala-OTcp et 1,29 g de HOBt. On ajuste le pH à 6-7 au papier pH, si nécessaire par NEM, et agite à TA durant 48 h. On évapore le DMF au bainmarie à 40° C sous une pression de 0,01 mm de mercure. On reprend l'huile résiduelle dans 50 ml d'AcOEt et lave successivement avec $KHSO_4$-$K_2SO_4$ 5 %, NaCl/$H_2O$, $NaHCO_3$ 5 %, NaCl/$H_2O$, sèche sur $MgSO_4$ et évapore le solvant. On reprend dans l'éther, un solide apparaît. Après 1 h au réfrigérateur, on essore et sèche.

Rendement : 1,46 9 (86 %) ; Fc : 117-120° C.

2. H-Ala-Sta-OMe.

On dissout 1,46 g de Z-Ala-Sta-OMe dans 20 ml de MeOH puis, à TA, on ajoute 1,03 g de formiate d'ammonium, agite jusqu'à dissolution, puis ajoute 400 mg de Pd/C 10 % et agite 5 min à TA. Après 5 min, un contrôle en CCM montre la disparition du produit de départ. Le pH est de 8 au papier pH. On filtre l'ensemble sur célite puis on filtre la solution méthanolique sur une colonne de résine Amberlite IR 45 (OH) et évapore le méthanol. On reprend dans l'éther et évapore à sec puis on dissout dans du $CH_2Cl_2$, filtre l'insoluble et évapore à sec pour obtenir une poudre blanche.

Rendement : 810 mg (75 %) ; Fc : 123-134° C.

3. Boc-Sta-Ala-Sta-OMe.

On dissout successivement dans 50 ml de $CH_2Cl_2$ à TA, 960 mg de H-Ala-Sta-OMe, 1 g de Boc-Sta-OH, 420 mg de HONSu, 750 mg de DCCI. Très rapidement, un précipité blanc apparaît. On agite à TA, après 7 h on filtre la DCU formée et lave celle-ci avec $CH_2Cl_2$. On lave la solution organique successivement avec $KHSO_4$-$K_2SO_4$ (5 % $H_2O$), $NaHCO_3$ (5 % $H_2O$). On sèche sur $MgSO_4$, filtre et évapore le solvant. Le produit est solubilisé dans le minimum de mélange chloroforme-MeOH (97,5/2,5, vol/vol) et déposé en tête d'une colonne de gel de silice ; on élue avec le même mélange et fractionne.

Un lot de produit pur et deux lots de produit à recycler dans les mêmes conditions sont recueillis, d'où un rendement global :

Rendement : 67 % ; Fc : 95-98° C.

4. H-Sta-Ala-Sta-OMe, TFA.

On traite à 0°C par 3,5 ml de TFA, 200 mg de Boc-Sta-Ala-Sta-OMe. On laisse agiter 20 min à TA. Puis après concentration sous vide, on épuise le résidu huileux par ajouts et concentrations successives d'éther anhydre. On obtient un solide blanc.

5. Boc-Met($O_2$)-Sta-Ala-Sta-OMe.

Sur le sel de TFA obtenu à l'étape précédente (85 mg), on ajoute 10 ml de DMF et ramène le pH à 7 par de la NMM. A -5°C, on introduit 56,3 mg de Boc-Met($O_2$)-OH, 45,9 mg de HOBt et 45,4 mg de DCCI. Après contrôle du pH, on laisse agiter 6 jours à TA. On ajoute alors 1 goutte d'AcOH sur le mélange réactionnel et laisse agiter 30min puis filtre la DCU ; le filtrat est repris par un mélange AcOH-butanol-1 à 75/25 en volume ; la phase organique est lavée successivement par une solution saturée de NaCl, par une solution à 1 % de $KHSO_4$, par de l'eau, par une solution à 1 % de $NaHCO_3$ et enfin à l'eau. Après séchage et concentration, on récupère 120 mg d'un résidu solide blanc que l'on chromatographie sur silice par AcOEt puis par un mélange AcOEt-MeOH à 9/1 en volume. On obtient 60 mg d'un solide blanc amorphe précipité dans l'hexane.

6. SR 42845.

On ajoute les 60 mg du produit obtenu à l'étape précédente à 2 ml de TFA à 0°C et on laisse agiter 20 min à TA. Après concentration sous vide, en épuise le résidu huileux par ajouts et concentrations successives d'éther anhydre. Sur le solide blanc de sel de TFA ainsi obtenu, on ajoute 10 ml de $CH_2Cl_2$ et ramène le pH à 7 par la NMM. On introduit alors 33,4 mg de Boc-Phe-ONp et 14 mg d'HOBt; après contrôle du pH, on laisse sous agitation 72 h à TA. La solution est diluée par ajouts de $CH_2Cl_2$ et la phase organique est lavée successivement par une solution saturée de ClNa, par une solution à 1 % de $KHSO_4$, par de l'eau, par une solution à 1 % de $NaHCO_3$, puis à l'eau. Après séchage et concentration, on récupère 58 mg d'un résidu solide que l'on chromatographie sur silice par AcOEt puis par un mélange AcOEt-MeOH à 9/1 en volume. On obtient 37 mg d'un solide blanc amorphe précipité dans l'hexane.

SPECTRE DE RMN

| Delta | Aspect | Protons | Attribution |
|---|---|---|---|
| 6,9-7,35 | 5 d, | | |
| 7,6-7,85 | J=8 Hz | 5 H | 5 NH |
| 8,05 | | | |
| 7,1-7,25 | m | 5 H | H ar (Phe) |
| 4,8-5 | m | 2 H | OH (Sta) |
| 2,9 | s | 2 H | $CH_3-SO_2$ |
| 3,45 | s | 3 H | $OCH_3$ |
| 1,25 | s | 9 H | $C(CH_3)_3$ |

Exemple 7

(R,S) Boc-Phe-Hph-Sta-Ala-Sta-OMe (SR 42727).

1. Boc-Ala-Sta-OMe.

4,1 g de H-Sta-OMe, TFA sont introduits dans 250 $cm^3$ de $CH_2Cl_2$; on ajoute successivement 1,7 $cm^3$ de NEM, 3,86 g de Boc-Ala-ONSu et 2 g de HOBt, puis on ajuste à pH 7 par addition de NEM et on agite une nuit à TA. Le mélange est lavé 2 fois par 500 $cm^3$ d'une solution saturée de $NaHCO_3$, 2 fois par 500 $cm^3$ d'une solution de $KHSO_4$-$K_2SO_4$ et par 500 $cm^3$ d'une solution saturée de ClNa puis séchée sur

$MgSO_4$ et évaporée à sec sous pression réduite.
Rendement : 3,86 g (74,7 %).

2. (R,S) Hph, HCl.

Le chlorhydrate d'homophénylalanine est préparé selon la méthode décrite dans J. Med. Chem., 1968, 11, 1258-1262.

3. (R,S) Boc-Hph, HCl.

Dans 50 ml d'eau, on mélange 2,8 g du chlorhydrate précédent et de la triéthylamine pour neutraliser à pH 7-8. On ajoute ensuite lentement, à environ 30-40° C, 25 ml de dioxanne dans lesquels on a préalablement dissous 3,12 g d'anhydride de tertbutyloxycarbonyl $(Boc)_2O$ puis on laisse sous agitation une nuit à TA. La solution obtenue est diluée à l'eau, lavée 2 fois à l'éther ; on ajoute AcOEt puis on amène progressivement le pH de la phase aqueuse vers 2,5-3 par addition d'$H_2SO_4$, 6N. Après 3 extractions par AcOEt et lavage de la phase organique à l'eau saturée de NaCl, on sèche sur $Na_2SO_4$, puis concentre. On récupère 1 g d'un solide blanc amorphe qui précipite dans l'hexane.

4. (R,S) Boc-Hph-Sta-OMe.

A 5 ml de TFA à 0° C, on ajoute 434 mg de Boc-Sta-OMe et laisse agiter 20 min à 0° C. Après concentration sous vide à TA, on épuise le résidu huileux par ajouts et concentrations successives d'éther anhydre. Au résidu solide blanc ainsi obtenu, on ajoute 15 ml de $CH_2Cl_2$ et ramène le pH à 7 par de la NMM, puis on ajoute 309,5 mg de DCCI, 229,5 mg d'HOBt et 419 mg de Boc-Hph, HCl précédemment obtenu. On vérifie le pH (pH 7) et laisse agiter 72 h à TA.

On filtre l'insoluble, et le filtrat chlorométhylénique est lavé successivement par une solution saturée de ClNa, une solution à 1 % de $KHSO_4$, de l'eau, une solution à 1 % de $NAHCO_3$, de l'eau. Après séchage et concentration, on obtient 900 mg d'un résidu huileux que l'on chromatographie sur silice fine par AcOEt. On obtient 230 mg d'un solide blanc amorphe précipité dans l'hexane.

5. (R,S) Boc-Hph-Sta-Ala-Sta-OMe.

On ajoute 182 mg de Boc-Ala-Sta-OMe obtenu à l'étape 1 à 4 ml de TFA à 0° C, on agite 30 min à 0° C, puis on concentre l'excès de TFA; le résidu huileux est ensuite épuisé par ajouts et concentrations successives d'éther anhydre. Il reste un résidu blanc auquel on ajoute 10 ml de $CH_2Cl_2$, le pH est amené à 7 par de la NMM, puis on ajoute 76,5 mg d'HOBt, 103, 2 mg de DCCI et 220 mg du produit obtenu à l'étape 4. On vérifie le pH (7) et laisse agiter 72 h à TA. L'insoluble est filtré, puis le filtrat est lavé par une solution saturée de NaCl, une solution à 1 % de $KHSO_4$, de l'eau, une solution à 1 % de $NaHCO_3$, de l'eau. Après séchage et concentration, on récupère 364 mg d'un résidu que l'on chromatographie sur silice fine par AcOEt. On obtient 190 mg d'un solide blanc amorphe qui est un mélange d'isomères.

6. SR 42727.

170 mg du produit obtenu à l'étape précédente sont ajoutés à 3 ml de TFA à 0° C ; après 30 min d'agitation à 0° C, on concentre l'excès de TFA et le résidu huileux est épuisé par ajouts et concentrations successives d'éther anhydre. Au résidu blanc obtenu, on ajoute 10 ml de $CH_2Cl_2$ et de la NMM pour amener le pH à 7, puis 96,6 mg de Boc-Phe-ONp et 38,3 mg d'HOBt ; on ajuste à pH 7 et laisse agiter 120 h à TA.

La solution est diluée par $CH_2Cl_2$ et la phase organique est lavée successivement par une solution de NaCl, une solution à 1 % de $KHSO_4$, de l'eau, une solution à 1 % de $NaHCO_3$, de l'eau. Après séchage et concentration, on récupère 300 mg d'un résidu que l'on chromatographie sur silice fine par AcOEt puis par un mélange AcOEt-MeOH à 9/1 en volume. On obtient 170 mg d'un solide blanc amorphe. C'est le produit attendu (SR 42727) sous forme d'un Mélange d'isomères au niveau de Hph.

SPECTRE DE RMN

| Delta | Aspect | Protons | Attribution |
|---|---|---|---|
| 7 - 8,1 | m | 15 H | 5 NH<br>10 H ar<br>(Phe, Hph) |
| 4,75 et<br>4,9 | d, J=4 Hz<br>J=4 Hz | 2 H | 2 OH (Sta) |
| 3,5 | s | 3 H | $OCH_3$ |
| 1,25 | s | 9 H | $C(CH_3)_3$ |

Exemple 8

Boc-Phe-(piMe)His-Sta-Ala-Sta-OMe (SR 43061).

Ce produit est préparé à partir de l'histidine protégée par 2 groupements Boc dont l'un est sur la fonction amine et l'autre sur l'azote télé de l'histidine :

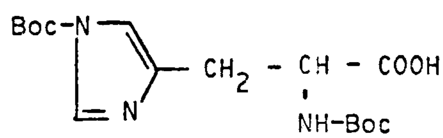

1. Bis(Boc)His-Sta-OMe.

3,7 g de Boc-Sta-OMe sont mis à réagir avec 15 ml de TFA entre 0° C et + 5° C pendant 20 min. On concentre, reprend 3 fois à l'éther anhydre et concentre au maximum. Le sel de TFA obtenu est placé dans 150 ml de $CH_2Cl_2$, amené à pH 7 par le DIPEA sous refroidissement. On ajoute alors 6,6 g de Bis-Boc-His sous forme de sel de DCHA, amène à pH 7 par DIPEA, rajoute 6,3 g de Bop et agite 3 jours à TA à pH 7. On lave à l'eau salée, à l'eau bicarbonatée puis à l'eau, sèche et concentre. Par chromatographie sur silice avec AcOEt comme éluant, on recueille 7,8 g de poudre blanche.

2. Boc-Phe-(piMe)His-Sta-OH.

On porte à reflux doux le mélange de 1 g du produit obtenu à l'étape précédente, 10 ml de DMF anhydre et 5 ml d'iodure de méthyle ; après 48 h de repos, on concentre sous vide de pompe. En reprenant à l'éther anhydre, on obtient une gomme jaune qui durcit dans AcOEt et se concrétise dans l'éther anhydre. On essore et sèche au pistolet. On ajoute la partie concrétisée et les jus mères concentrés à 20 ml de TFA à 0° C. On agite 20 min, concentre, traite à l'éther anhydre 3 fois et concentre au maximum. Le sel de TFA obtenu est dissous dans 25 ml de $CH_2Cl_2$ et amené à pH 7 avec de la NMM. On y ajoute 724,5 mg de Boc-Phe-ONp et 299 mg d'HOBt et on agite 4 jours à TA. On lave à l'eau salée, à l'eau bicarbonatée puis à l'eau, sèche et concentre. Par chromatographie sur silice et élution par AcOEt-MeOH à 8/2 en volume, on recueille 2 fractions polaires concrétisées dans l'éther anhydre. On obtient 639 mg de produit. On effectue ensuite une saponification à partir de 300 mg auxquels on ajoute 13 ml de MeOH, 2 ml d'eau, 167 mg de baryte hydratée puis, après 1 h, 84 g de baryte hydratée. L'ester a disparu en CCM. On ajoute des petits morceaux de carboglace jusqu'a pH inférieur à 7, on essore sur célite, rince au méthanol, concentre le filtrat ; le résidu est repris avec le minimum de MeOH tiède, filtré et concentré, on obtient ainsi 248 mg de poudre blanche.

3. SR 43061.

Le composé obtenu à l'étape précédente est couplé avec Boc-Ala-Sta-OMe en opérant selon les méthodes précédemment décrites.

SPECTRE DE RMN

| Delta | Aspect | Protons | Attribution |
|---|---|---|---|
| 6,9, 7,4<br>7,6 7,8,<br>8,05 | 5 d,<br>J=8 Hz | 5 H | NH |
| 7,1-7,25 | m | 5 H | 5 H ar (Phe) |
| 6,7, 7,4 | 2 s | 2 H | 2 H im |
| 3,5 | s | 3 H | $OCH_3$ |
| 3,55 | s | 3 H | $(piCH_3)His$ |
| 1,25 | s | 9 H | $C(CH_3)_3$ |

Exemple 9

Boc-Phe-(tauMe)His-Sta-Ala-Sta-OMe (SR 43062).

1. La (tauMe)His OH est préparée selon la méthode décrite dans J. R. Neth. Chem. Soc., 1978, 97, 293-295.

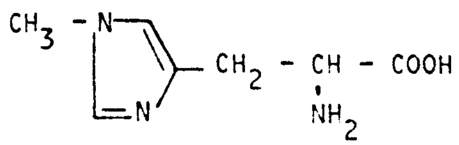

2. (tauMe)His-OMe, 2 HCl.

4,5 g du dichlorhydrate du produit précédent sont dissous dans 50 ml de MeOH ; on sature par HCl gazeux à 10-15 °C puis laisse sous agitation à TA pendant 72 h. On concentre à sec, reprend à l'éther 2 fois puis le résidu est repris dans l'acétone, il cristallise, après essorage, on obtient 4 g.

3. Boc-Phe-(tauMe)His-OMe.

508 mg du produit précédent sont placés dans 25 ml de $CH_2Cl_2$ et amenés à pH 7 par addition de DIPEA. On ajoute 772 mg de Boc-Phe-ONp et 306 mg d'HOBt. Après 2 jours d'agitation à TA, on lave à l'eau glacée puis sèche et concentre. Par chromatographie sur silice et élution par un mélange AcOEt-MeOH à 9/1 en volume, on recueille 849 mg du produit attendu.

4. Boc-Phe-(tauMe)His.

768 mg du produit précédent sont dissous dans 33 ml de MeOH et 5 ml d'eau auxquels on ajoute 561

mg de baryte hydratée. Après 1 h, on ajoute 280 mg de baryte et laisse encore 1 h sous agitation. On additionne alors des petits morceaux de carboglace jusqu'à ce que le pH soit inférieur à 7 puis on essore sur célite, rince par MeOH et concentre. On reprend par MeOH, filtre le léger insoluble et concentre. On reprend à l'éther puis essore pour obtenir 634 mg de produit blanc.

5. H-Sta-Ala-Sta-OMe, TFA.

Il est préparé selon la méthode décrite à l'exemple 1 à partir de 130 mg de Boc-Sta-Ala-Sta-OMe.

6. Boc-Phe-(tauMe)His-Sta-Ala-Sta-OMe.

Au sel de TFA précédemment obtenu placé dans 10 ml de $CH_2Cl_2$, on ajoute du DIPEA pour amener le pH à 7; puis on ajoute 124 mg de Bop et 104 mg du produit obtenu à l'étape 4, on ajoute 3 ml de DMF anhydre et laisse agiter 72 h à pH 7. On lave à l'eau salée puis au bicarbonate dilué et à l'eau, on sèche et concentre. On effectue une chromatographie sur silice en éluant par un mélange AcOEt-MeOH à 9/1 en volume. On isole une fraction de 80 mg de produit blanc.

SPECTRE DE RMN

| Delta | Aspect | Protons | Attribution |
|---|---|---|---|
| 7,0, 7,3<br>7,4, 7,95<br>8,1 | 5 d,<br>J=8 Hz<br>J=10 Hz<br>J=6 Hz<br>J=6 Hz<br>J=8 Hz | 5 H | 5 NH |
| 6,8 et 7,4 | 2 s | 2 H | 2 H im |
| centré à 4,95 | m | 2 H | 2 OH (Sta) |
| 7,1-7,3 | m | 5 H | 5 H ar (Phe) |
| 3,5 | d | 6 H | $OCH_3$ (tauMe) His |
| 1,25 | s | 9 H | $C(CH_3)_3$ |

Exemple 10

Boc-Phe-(cyclohexyl)Gly-Sta-Ala-Sta-OMe (SR 43091).

La N-Boc(2-alpha cyclohexyl)glycine est préparée à partir de L-alpha phényl glycine par hydrogénation catalytique sur catalyseur d'Adams (Pt $O_2$). On opère ensuite selon les méthodes précédemment décrites pour préparer le SR 43091.

SPECTRE DE RMN

| Delta | Aspect | Protons | Attribution |
|---|---|---|---|
| 6,9-7,85 | m | 5 H | 5 NH |
| centré à 7,2 | m | 5 H | 5 H ar |
| 4,8 et 4,95 | 2 d J=5 Hz J=4 Hz | 2 H | 2 OH (Sta) |
| 3,5 | s | 3 H | $OCH_3$ |
| 1,25 | s | 9 H | $C(CH_3)_3$ |

Exemple 11

Boc-Phe-(Pyr-3)Ala-Sta-Ala-Sta-OMe (SR 43429).

1. Acétamido-2 éthoxycarbonyl-2 (pyridyl-3)-3 propionate d'éthyle.

A 11 g d'une solution de sodium dans 300 ml de EtOH, on ajoute 48 g d'acétamidomalonate de diéthyle puis, en portant à reflux, on additionne 36 g de chlorhydrate de chlorométhyl-3 pyridine, on conserve 5 h à reflux. On évapore EtOH, reprend par l'eau, extrait, sèche, concentre et chromatographie sur silice. Le mélange $CH_2Cl_2$-AcOEt,à 60/40 en volume, élue 23 g du composé attendu.

2. (Pyr-3)Ala, 2 HCl.

15 g du diester précédent sont portés à reflux dans 200 ml d'HCl 5 N pendant 5 h. On concentre à sec, ajoute EtOH, évapore. Le procédé est réitéré jusqu'à recristallisation du dichlorhydrate : 15 g.

3. Boc(Pyr-3)Ala.

1 g du dichlorhydrate précédent est dissous dans un mélange à volume égal eau-dioxanne. On ajoute 350 mg de soude et 1 g de $(Boc)_2O$ puis on agite une nuit à TA. Le dioxanne est évaporé, on ajoute de l'eau, neutralise par 8 g d'Amberlite CG 120 I (forme acide), agite 1 h à TA puis le milieu est mis sur une colonne contenant 8 g de la même résine. On élue par de l'eau distillée jusqu'à obtenir pH 5 puis élue par une solution d'ammoniaque à 4 % dans l'eau distillée. Les eaux sont concentrées. On recueille 0,69 g du produit attendu.

4. Boc-(Pyr-3)Ala-Sta-OMe.

On agite pendant 3 jours à TA dans 10 ml d'acétonitrile un mélange de 465 mg de Sta-OMe, TFA, 500 mg de Boc-(Pyr-3)Ala en présence de 780 mg de Bop, le milieu étant maintenu vers pH 7 par addition de DIPEA. On évapore alors à siccité, ajoute de l'eau, extrait par AcOEt, lave par une solution de $Na_2CO_3$, de l'eau, de l'eau saline, puis on sèche sur $MgSO_4$ et évapore à siccité. Le résidu est chromatographié sur gel de silice par un mélange éluant $CH_2Cl_2$-AcOEt à 1/9 en volume qui élue successivement :
- l'isomère A le moins polaire : 150 mg ;
- l'isomère B le plus polaire : 180 mg.

5. SR 43429.

On obtient le composé attendu sous forme d'un isomère pur à partir de l'isomère B en utilisant les méthodes de couplage habituelles.

SPECTRE DE RMN

| Delta | Aspect | Protons | Attribution |
|---|---|---|---|
| 8,41 | s | 1 H | $H_2$ (Pyr) |
| 8,32 | d, J=4 Hz | 1 H | $H_6$ (Pyr) |
| 8,02-7,33 | m | 6 H | NHCO, H (Pyr) |
| 7,27-7,04 | m | 5 H | $C_6H_5$ |
| 6,87 | d, J=8 Hz | 1 H | NH $CO_2$ |
| 4,97 | d, J=4 Hz | 1 H | OH |
| 4,85 | d, J=4 Hz | 1 H | OH |
| 4,64-3,90 | m | 3 H | CH alpha ((Ala, Phe, (Pyr-3) Ala) |
| 3,90-3,67 | m | 4 H | CHNH, CHOH (Sta) |
| 3,50 | s | 3 H | $OCH_3$ |
| 3,10-2,47 | m | 4 H | $CH_2C_5H_4N$ $CH_2C_6H_5$ |
| 2,96-1,90 | m | 4 H | $CH_2CO$ (Sta) |
| 1,57-1,00 | m | 18 H | $(CH_3)_3C$, $CH_3$ (Ala) CH, CH (Sta) |
| 0,87-0,66 | m | 12 H | $CH_3$ (Sta) |

En utilisant les mêmes méthodes de couplage, on a préparé les composés suivants :

| N° SR | Formule |
|---|---|
| 42823 | Boc-Phe-Phg-Sta-Ala-Sta-OMe |
| 42928 | Boc-Phe-Asn-Sta-Ala-Sta-OMe |
| 42939 | Boc-Phe-(Ph $CH_2O$)Ser-Sta-Ala-Sta-OMe |
| 42980 | Boc-Phe-Gln-Sta-Ala-Sta-OMe |
| 43075 | (R,S) Boc-Phe-(thiényl-2)Gly-Sta-Ala-Sta-OMe |
| 43266 | Boc-Phe-(Ph)Gln-Sta-Ala-Sta-OMe |
| 43281 | Boc-Phe-(Ph)Asn-Sta-Ala-Sta-OMe |
| 43297 | Boc-Phe-($(CH_3)_3CO$)Ser-Sta-Ala-Sta-OMe |
| 43365 | Boc-Phe-($CH_3CH_2O$)Ser-Sta-Ala-Sta-OMe |
| 43366 | Boc-Phg-Phg-Sta-Leu-Phe-OMe |
| 43391 | Boc-Phe-(tauEt)His-Sta-Ala-Sta-OMe |
| 43428 | Boc-Phe-((méthyl-1) imidazolyl-2) Ala-AHPPA-Ala-Sta-$OCH_3$ |
| 43554 | Boc-Phe-Cpg-Sta-Ala-Sta-OMe |
| 43555 | Boc-Phe-((Pyr-2)$CH_2O$) Ser-Sta-Ala-Sta-OMe |
| 43556 | Boc-Phe-(cyclohexyl) Ala-Sta-Ala-Sta-OMe |
| 43761 | iVa-Phe-(tauMe)His-Sta-Ala-Sta-OH |

Ces produits sont caractérisés par leur spectre de RMN.

SPECTRE DE RMN DU SR 42823

| Delta | Aspect | Protons | Attribution |
|---|---|---|---|
| 7-8,3 | m | 15 H | 5 NH<br>10 H ar |
| 4,8 et 5 | 2 d,<br>J=4 Hz | 2 H | 2 OH (Sta) |
| 3,5 | s | 3 H | $OCH_3$ |
| 1,2 | s | 9 H | $C(CH_3)_3$ |

SPECTRE DE RMN DU SR 42928

| Delta | Aspect | Protons | Attribution |
|---|---|---|---|
| 6,85-8,25 | m | 12 H | 5 NH<br>5 H ar $CONH_2$ |
| 5,8 et 5,9 | 2 d,<br>J=6 Hz | 2 H | OH (Sta) |
| 1,25 | s | 9 H | $C(CH_3)_3$ |

SPECTRE DE RMN DU SR 42939

| Delta | Aspect | Protons | Attribution |
|---|---|---|---|
| 6,9-8,1 | m | 15 H | 5 NH<br>10 H ar |
| 4,85 et<br>4,95 | 2 d,<br>J=5 Hz | 2 H | OH (Sta) |
| 4,45 | s | 2 H | $O\underline{CH_2}C_6H_5$ |
| 3,5 | s | 3 H | $OCH_3$ |
| 1,25 | s | 9 H | $C(CH_3)_3$ |

SPECTRE DE RMN DU SR 42980

| Delta | Aspect | Protons | Attribution |
|---|---|---|---|
| 6,75-8 | m | 12 H | 5 NH<br>5 H ar<br>$NH_2$ (Gln) |
| 4,85 et<br>4,95 | 2 d,<br>J=5 Hz | 2 H | OH (Sta) |
| 3,5 | s | 3 H | $OCH_3$ |
| 1,25 | s | 9 H | $C(CH_3)_3$ |

SPECTRE DE RMN DU 43075

| Delta | Aspect | Protons | Attribution |
|---|---|---|---|
| 6,9-8,5 | m | 13 H | 5 NH<br>5 H ar 3 H<br>thiophéniques |
| 4,8-4,9 | m | 2 H | OH (Sta). |
| 3,5 | s | 3 H | $OCH_3$ |
| 1,25 | s | 9 H | $C(CH_3)_3$ |

SPECTRE DE MRN DU SR 43266

| Delta | Aspect | Protons | Attribution |
|---|---|---|---|
| 9,8 | s | 1 H | NH (NH $C_6H_5$) |
| 6,9-9,1 | m | 15 H | 5 NH 10 H ar |
| 4,85-5 | m | 2 H | OH (Sta) |
| 3,5 | s | 3 H | $OCH_3$ |
| 1,25 | s | 9 H | $C(CH_3)_3$ |

SPECTRE DE RMN DU SR 43281

| Delta | Aspect | Protons | Attribution |
|---|---|---|---|
| 10,1 | s | 1 H | NH (NH-$C_6H_5$) |
| 6,95-8,4 | m | 15 H | 5 NH<br>10 H ar |
| 4,8 et<br>4,95 | 2 d,<br>J=6 Hz | 2 H | 2 OH (Sta) |
| 3,8 | s large | 2 H | 2 H alpha (Sta) |
| 3,5 | s | 3 H | $CH_3$ ester |
| 1,25 | s | 9 H | $C(CH_3)_3$ |

SPECTRE DE RMN DU SR 43297

| Delta | Aspect | Protons | Attribution |
|---|---|---|---|
| 7 à 7,85 | m | 10 H | 5 NH 5 ar |
| 4,6 à 5 | m | 2 H | OH (Sta) |
| 3,8 | s large | 2 H | 2 H alpha (Sta) |
| 3,5 | s | 3 H | $CH_3$ ester |
| 1,25 | s | 9 H | Boc |
| 1,05 et 1 | 2 pics isomères | 9 H | $C(CH_3)_3$ |

SPECTRE DE RMN DU SR 43365

| Delta | Aspect | Protons | Attribution |
|---|---|---|---|
| 7,2 | m | 5 H | ar |
| (6,95<br>(7,30<br>(7,45<br>(7,80<br>(8,0 | 5 d, J=8 Hz | . | 5 H NH |
| 4,8-4,95 | 2 d, J=4 Hz | 2 H | OH (Sta) |
| (3,75<br>(4,2<br>(4,4 | 3 m | 4 H | 4 H alpha (Phe, Ser, Sta) |
| 3,5 | s | 3 H | $OCH_3$ |
| 3,35 | q | | $OC\underline{H_2}CH_3$ |
| 1,25 | s | 9 H | $C(CH_3)_3$ |
| 1,0 | t | 3 H | $OCH_2C\underline{H_3}$ . |

SPECTRE DE RMN DU SR 43391

| Delta | Aspect | Protons | Attribution |
| --- | --- | --- | --- |
| 6,8 à 8,2 | m | 12 H | 5 NH, 5 ar,<br>2 His |
| 4,95 | m | 2 H | OH (Sta) |
| 3,5 | s | 3 H | $OCH_3$ ester |
| 1,25 | s | 9 H | $C(CH_3)_3$ |

SPECTRE DE RMN DU SR 43366

| Delta | Aspect | Protons | Attribution |
| --- | --- | --- | --- |
| 7,1 à 8,75 | m | 15 H | 10 H $C_6H_5$,<br>5 NH |
| 5,35 et<br>5,5 | 2 d,<br>J=8 Hz | 2 H | 2 H alpha<br>(Phg) |
| 5 | m | 1 H | OH (Sta) |
| 3,65 | m | 2 H | H alpha (Sta) |
| 3,5 | s | 3 H | $OCH_3$ ester |
| 1,35 | s | 9 H | $C(CH_3)_3$ |

SPECTRE DE RMN DU SR 43428 (2 isomères : 7/3).

| Delta | Aspect | Protons | Attribution |
|---|---|---|---|
| 8,25-7,76 | m | 3 H | NH CO |
| 7,39 | d, J=8 Hz | 1 H | NH CO |
| 7,25-7,00 | m | 10 H | $C_6H_5$ |
| 7,00-6,89 | m | 2 H | $NHCO_2$, H im |
| 6,89)<br>5,71) | 2 s | 1 H | H im |
| 5,32-4,0 | m | 5 H | OH, H alpha (Ala, Phe, im) |
| 4,0-3,67 | m | 4 H | CHNH, CHOH (AHPPA, Sta) |
| 3,53)<br>3,44) | 2 s | 3 H | $N-CH_3$ |
| 3,50 | s | 3 H | O $CH_3$ |
| 3,00-1,95 | m | 10 H | $\underline{CH_2}-C_6H_5$, $CH_2$ im, $CH_2CO$ (AHPPA, Sta) |
| 1,57-1,00 | m | 15 H | $(CH_3)_3C$, $CH_3$ (Ala), CH et $CH_2$ (Sta) |
| 0,77-0,69 | m | 6 H | $CH_3$ (Sta) |

<u>SPECTRE DE RMN DU SR 43554</u>

| Delta | Aspect | Protons | Attribution |
|---|---|---|---|
| 6,92-7,89 | m dont 4 d à 6,95 7,37, 7,57 7,89 ppm J=6 Hz | 10 H | 5 NH (amides) 5 ar (Phe) |
| 4,95 4,80 | 2 d J=4 Hz | 2 H | 2 OH (Sta) |
| 3,35-4,22 | m dont s à 3,51 ppm ($OCH_3$ Sta) | 10 H | CH alpha (Phe) CH alpha (Cpg) 4 CH alpha (Sta) CH alpha (Ala) $OCH_3$ Sta OMe |
| 2,03-2,95 | m | 6 H | $CH_2$ (Phe) 2 $CH_2$-CO (Sta) |
| 0,68-1,6 | m dont s à 1,20 ppm | 32 H | 4 $CH_3$ (Sta) $CH_3$ (Ala) $C(CH_3)_3$ 4 $CH_2$ (cyclopentyl) |

<u>SPECTRE DE RMN DU SR 43556</u>

| Delta | Aspect | Protons | Attribution |
|---|---|---|---|
| 6,90-8,0 | m dont 3 d à 6,93 7,87, 7,96 J=4 Hz | 10 H | 5 NH (amides) 5 ar (Phe) |
| 4,92 et 4,83 | 2 d J=4 Hz | 2 H | 2 OH (Sta) |
| 3,5-4,38 | m dont s à 3,5 | 10 H | CH alpha (Phe) CH alpha (Ala) CH alpha ((cyclo-hexyl)Ala) 4 CH alpha (Sta) $CH_3$ (OMe) |
| 2-2,91 | m | 6 H | $CH_2$ (Phe) 2 $CH_2CO$ (Sta) |
| 0,94-1,74 | m | 31 H | 3 $CH_3$ (Boc) 13 $\underline{CH_2}-C_6H_{11}$ 2 $CH_2$-CH (Sta) $CH_3$ (Ala) |
| 0,71-0,94 | m | 12 H | 4 $CH_3$ (2 Sta) |

<u>SPECTRE DE RMN DU SR 43761</u>

| Delta | Aspect | Protons | Attribution |
|---|---|---|---|
| 6,81-8,52 | m dont 3 d centrés à 7,03, 8,26 8,5 J=8 Hz | 12 H dont 3 H | 5 ar ; 5 NH ; 1 H (His) NH (amides) |
| | s à 7,4 | 1 H | H (His) |
| | s à 7,22 | 5 H | H Ar |
| | s à 6,81 | 1 H | H (His) |
| 5,13 | m | 1 H | OH (Sta) |
| 4,15-4,44 | m | 3 H | H alpha (Phe) H alpha (His) H alpha (Ala) |
| 3,58-3,78 | m | 4 H | Sta |
| 3,52 | s | 3 H | $CH_3$ (tauMe)His |
| 2,63-3,04 | m | 4 H | $CH_2$ (Phe) $CH_2$ (His) |
| 0,97-2,11 | m | 19 H | 2 $CH_2CO$ (Sta) $CH_2$-CH (iVa) 2 $CH_2$-CH (Sta) $CH_3$ (Ala) |
| 0,55-0,82 | m | 16 H | 2 $CH_3$ (iVa) 4 $CH_3$ (Sta) |

Les produits selon l'invention ont été étudiés en ce qui concerne leurs propriétés thérapeutiques et notamment leur action inhibitrice enzymatique. Plus particulièrement, les composés ont été évalués "in vitro" sur l'inhibition de l'Activité Rénine Plasmatique Humaine (A.R.P.) et les résultats sont supérieurs à ceux obtenus avec le produit naturel, la pepstatine.

I - METHODE.

Le méthode d'évaluation est inspirée de GUYENE (J. Clin. Endocrinol. Metab., 1976, 43, 1301), dans la mesure où l'inhibition de l'A.R.P. est évaluée à partir d'un pool de plasmas humains, riche en rénine (15 à 20 mg d'angiotensine I libérée par millilitre et par heure) incubée pendant 60 min à 37° C, dans un tampon phosphate à pH 7,4, en présence de concentrations croissantes du produit à étudier.

Le plasma humain contient le substrat : l'angiotensinogène et l'enzyme : la rénine. L'angiotensine I libérée en cours de réaction est mesurée par dosage radio-immunologique à l'aide d'un kit : Plasma Renin Activity Kit de Travenol (n° CA 533553). Un inhibiteur de l'enzyme de conversion, le fluorure de phénylméthylsulfonyle (PMSF) est ajouté au milieu d'incubation. Le volume total d'incubation est de 555 microlitres répartis ainsi :

- 420 microlitres de plasma humain

- 11 à 50 microlitres du produit à étudier à des concentrations variables ;
- 119 à 80 microlitres de tampon phosphate ;
- 5 microlitres de PMSF.

On prépare une solution d'acide acétique dans le méthanol (19/1 en volume) et une solution de soude dans le méthanol (2/1 en volume). Dans un mélange équivolumique de ces 2 solutions, on prépare une solution mère du peptide à 0,001 M. Les dilutions ultérieures du peptide sont alors effectuées dans le tampon phosphate.

La quantité de solvant présente dans une solution du peptide à une concentration inférieure à 0,0001 M n'interfère pas avec les résultats.

II - RESULTATS.

Les résultats sont exprimés par la dose de composé évaluée en moles, qui inhibe de 50 % ($IC_{50}$) l'Activité Rénine Plasmatique Humaine observée en l'absence d'inhibiteur.

Les résultats obtenus avec divers produits de l'invention sont représentés dans le tableau (I) suivant où figurent les $IC_{50}$ de chaque molécule en ce qui concerne leur inhibition de l'Activité Rénine Plasmatique Humaine à pH 7,4. De 5 à 10 doses ont été nécessaires pour déterminer ces $IC_{50}$. La pepstatine, en tant que substance de référence, est toujours testée en parallèle dans chaque expérience. Les résultats sont exprimés par leur valeur logarithmique (-Log $IC_{50}$).

Tableau I

| N° SR | Inhibition A.R.P. Humaine -Log $IC_{50}$ M |
|---|---|
| | pH 7,4 |
| Pepstatine | 4,92 |
| 42 823 | 7,03 |
| 42 926 | 6,40 |
| 42 928 | 5,92 |
| 42 939 | 6,76 |
| 42 980 | 6,50 |
| 43 059 | 5,52 |
| 43 064 | 6,50 |
| 43 075 | 6,60 |
| 43 144 | 5,88 |
| 43 150 | 5,21 |

| | |
|---|---|
| 43 151 | 6,66 |
| 43 281 | 5,85 |
| 43 297 | 5,67 |
| 43 365 | 6,95 |
| 43 391 | 7,19 |
| 43 428 | 6,16 |
| 43 429 | 6,38 |
| 43 554 | 7,43 |
| 43 555 | 6,50 |
| 43 556 | 6,31 |
| 43 761 | 6,82 |

La toxicité des produits selon l'invention est compatible avec leur utilisation en thérapeutique.

## Revendications

**Revendications pour les Etats contractants suivants : BE, CH, FR, GB, IT, LI, LU, NL, SE**

**1.** Dérivé peptidique :

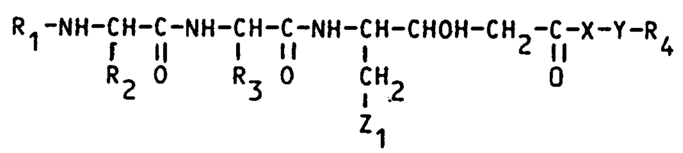

(1)

dans laquelle

$R_1$ représente un groupe acyle choisi parmi les groupes alkylcarbonyle, alcoxycarbonyle, arylcarbonyle, arylalkylcarbonyle, arylalcoxycarbonyle, hétérocyclylcarbonyle, hétérocyclylalkylcarbonyle dans lequel le groupe alkyle désigne les radicaux d'hydrocarbures aliphatiques saturés, ou insaturés contenant 1 à 10 atomes de carbone, ledit groupe alkyle étant éventuellement substitué par un groupe hydroxy, hétérocyclylcarbonylalkylcarbonyle, hétérocyclylalcénylcarbonyle, cycloalkylcarbonyle ou un groupe (alkyle contenant jusqu'à 6 atomes de carbone)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle contenant jusqu'à 6 atomes de carbone ;

$R_2$ représente un groupe alkyle contenant jusqu'à 6 atomes de carbone, non substitué ou substitué par un phényle, naphtyle, cyclohexyle, ou pyridyle ou $R_2$ représente un radical phényle, naphtyle, cyclohexyle ou pyridyle ;

$R_3$ représente un alcényle contenant jusqu'à 6 atomes de carbone, un phényle, un naphtyle, un cycloalkyle contenant 3 à 6 atomes de carbone, un groupe hétérocyclique monocyclique à 5 ou 6 chaînons non substitué ou substitué par un alkyle contenant jusqu'à 6 atomes de carbone ou trifluorométhyle, choisi parmi la pyrrolidine, l'imidazole, le thiazole, le thiophène, le furanne, le pyrrole, le triazole, l'oxazole, l'isoxazole, la pyridine, les thiadazoles; ou bien un alkyle contenant jusqu'à 6 atomes de carbone substitué par un groupe di(alkyle contenant jusqu'à 6 atomes de carbone)-amino, par un carboxyle estérifié par un alkyle contenant jusqu'à 6 atomes de carbone ou un benzyle, par un carbamoyle libre ou substitué par un

ou deux alkyles contenant jusqu'à 6 atomes de carbone ou par un phényle, par un groupe alcoxy contenant jusqu'à 6 atomes de carbone ou benzyloxy, par un groupe pyridylméthyloxy, par un groupe (alkyle contenant jusqu'à 6 atomes de carbone) sulfinyle ou (alkyle contenant jusqu'à 6 atomes de carbone) sulfonyle, par un cycloalkyle contenant de 3 à 6 atomes de carbone, ou par un groupe hétérocyclique non substitué ou substitué avec un alkyle contenant jusqu'à 6 atomes de carbone ou un trifluorométhyle choisi parmi la pyrrolidine, l'imidazole, le thiazole, le thiophène, le furanne, le pyrrole, le triazole, l'oxazole, l'isoxazole, la pyridine, les thiadiazoles, à la condition que $R_3$ ne soit pas le groupe (imidazolyl-4)-méthyle.

$R_4$ représente un hydroxyle, un alcoxy contenant jusqu'à 6 atomes de carbone, un benzyloxy ou un groupe amino libre ou substitué par 1 ou 2 alkyles contenant jusqu'à 6 atomes de carbone.

$Z_1$ représente l'isopropyle, le phényle ou le cyclohexyle formant respectivement avec le radical

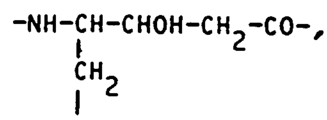

le résidu de l'aminoacide statine, à savoir, l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA) ;

X-Y est un dipeptide choisi parmi Ala-Sta, Ala-Leu, Leu-Phe, Val-Sta, Abu-Sta, Ile-Ser, Phg-Sta ;

ainsi que les sels éventuels pharmaceutiquement acceptables dudit dérivé peptidique avec les acides minéraux ou organiques ou des métaux alcalins ou alcalino-terreux.

**2.** Dérivé peptidique selon la revendication 1, caractérisé en ce que $R_1$ représente l'un des groupements suivants :

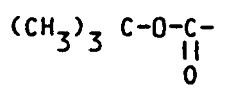

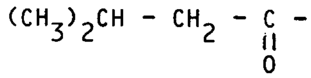

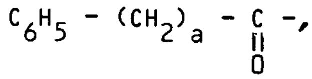, où a = 0, 1 ou 2

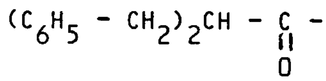

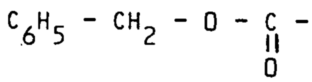

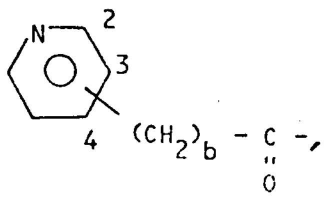, où n = 0,1,2,3,4,5 ou 6

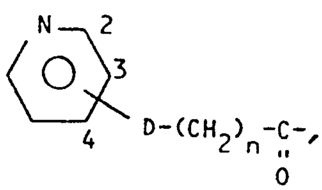, où D = 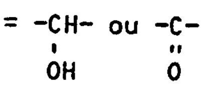

et n = 1,2,3,4 ou 5

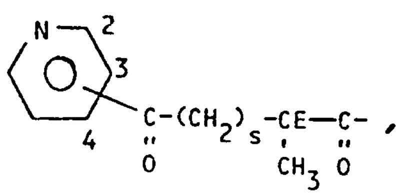, où E = $-H$ ou $-CH_3$

et s = 1,2,3 ou 4

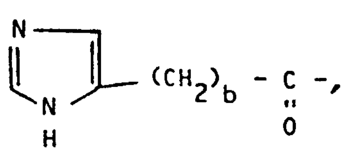, où n = 0,1,2,3,4,5 ou 6

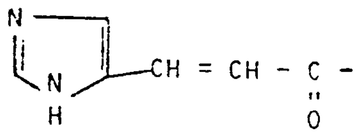

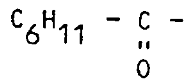

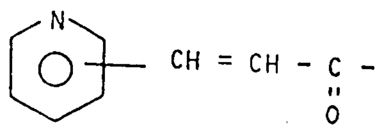

$NH_2CH_2CH_2$ - $SO_2$ -
$BocNHCH_2CH_2$ - $SO_2$ -
Z-$NHCH_2CH_2$ - $SO_2$ -
A-$SO_2$ - où A est un alkyle inférieur
$C_6H_5$ - $(CH_2)_a$ - $SO_2$, où a = 0,1 ou 2,
ou un des sels éventuels pharmaceutiquement acceptables dudit dérivé peptidique avec les acides minéraux ou organiques ou avec des métaux alcalins ou a alcalino-terreux.

**3.** Dérivé peptidique selon la revendication 1, caractérisé en ce que $R_1$ est choisi parmi les groupements suivants :
- isovaléryle,
- (pyridyl-2)-4 oxo-4 butyryle,
- (pyridyl-2)-4 hydroxy-4 butyryle,
- (pyridyl-3)-3 propionyle,
- (pyridyl-3)-4 butyryle,
- nicotinoyle,
- benzène sulfonyle.

**4.** Dérivé peptidique selon la revendication 1, caractérisé en ce que le résidu -NH-CH($R_3$)-CO- représente le résidu (tauMe)His.

**5.** Procédé de préparation d'un dérivé peptidique selon la revendication 1, caractérisé en ce qu'à partir d'un composé de formule : H-Y-$R'_4$, dans laquelle $R'_4$ représente un alcoxy contenant jusqu'à 6 atomes de carbone, un benzyloxy ou un groupe amino libre ou substitué par 1 ou 2 alkyles contenant jusqu'à 6 atomes de carbone et Y est choisi parmi les résidus des acides aminés Sta, Leu, Phe et Ser, on couple, étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant soit un ester activé de l'aminoacide à coupler, soit l'aminoacide M-protégé en présence de dicyclohexylcarbodiimide, en ce qu'éventuellement on saponifie le composé obtenu pour former le peptide de formule I dans laquelle $R_4$ = OH et/ou en ce qu'éventuellement on transforme le peptide obtenu en l'un de ses sels pharmaceutiquement acceptables avec des acides minéraux ou organiques ou des métaux alcalins ou alcalino-terreux.

**6.** Compositions pharmaceutiques, caractérisées en ce qu'elles contiennent un produit selon la revendication 1 en tant qu'ingrédient actif.

**7.** Compositions pharmaceutiques selon la revendication 6, utilisables notamment pour le traitement de la tension artérielle, caractérisées en ce qu'elles contiennent de 1 à 1000 mg de produit selon la revendication 1 par unité de dosage en mélange avec un excipient pharmaceutique.

**Revendications pour l'Etat contractant suivant : AT**

**1.** Procédé pour l'obtention de dérivés peptidiques répondant à la formule :

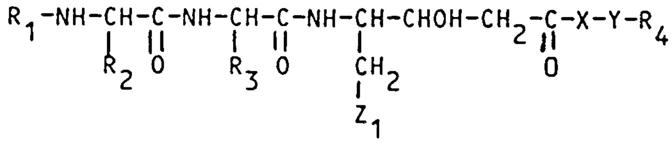 (1)

dans laquelle

- $R_1$ représente un groupe acyle choisi parmi les groupes alkylcarbonyle, alcoxycarbonyle, arylcarbonyle, arylalkylcarbonyle, arylalcoxycarbonyle, hétérocyclylcarbonyle, hétérocyclylalkylcarbonyle dans lequel le groupe alkyle désigne les radicaux d'hydrocarbures aliphatiques saturés ou insaturés contenant 1 à 10 atomes de carbone, ledit groupe alkyle étant éventuellement substitué par un groupe hydroxy, hétérocyclylcarbonylalkylcarbonyle, hétérocyclylalcénylcarbonyle, cycloalkylcarbonyle ou un groupe (alkyle contenant jusqu'à 6 atomes de carbone)-sulfonyle non substitué ou substitué sur l'alkyle par un groupe amino libre ou portant un groupe protecteur ou par un phényle ; ou un groupe phénylsulfonyle non substitué ou substitué sur le noyau phényle par un alkyle contenant jusqu'à 6 atomes de carbone ;
- $R_2$ représente un groupe alkyle contenant jusqu'à 6 atomes de carbone non substitué ou substitué par un phényle, naphtyle, cyclohexyle, ou pyridyle ou $R_2$ représente un radical phényle, naphtyle, cyclohexyle ou pyridyle ;
- $R_3$ représente un alcényle contenant jusqu'à 6 atomes de carbone, un phényle, un naphtyle, un cycloalkyle contenant 3 à 6 atomes de carbone, un groupe hétérocyclique monocyclique à 5 ou 6 chaînons non substitué ou substitué par un alkyle contenant jusqu'à 6 atomes de carbone ou trifluorométhyle choisi parmi la pyrrolidine, l'imidazole, le thiazole, le thiophène, le furanne, le pyrrole, le triazole, l'oxazole, l'isoxazole, la pyridine, les thiadiazoles ; ou bien un alkyle contenant jusqu'à 6 atomes de carbone substitue par un groupe di(alkyle contenant jusqu'à 6 atomes de carbone)-amino, par un carboxyle estérifié par un alkyle contenant jusqu'à 6 atomes de carbone ou un benzyle, par un carbamoyle libre ou substitué par un ou deux alkyles contenant jusqu'à 6 atomes de carbone ou par un phényle, par un groupe alcoxy contenant jusqu'à 6 atomes de carbone ou benzyloxy, par un groupe pyridylméthyloxy, par un groupe (alkyle contenant jusqu'à 6 atomes de carbone)sulfinyle ou (alkyle contenant jusqu'à 6 atomes de carbone)sulfonyle, par un cycloalkyle contenant de 3 à 6 atomes de carbone, ou par un groupe hétérocyclique monocyclique à 5 ou 6 chaînons non substitué ou substitué avec un alkyle contenant jusqu'à 6 atomes de carbone ou un trifluorométhyle, choisi parmi la pyrrolidine, l'imidazole, le thiazole, le thiophène, le furanne, le pyrrole, le triazole, l'oxazole, l'isoxazole, la pyridine, les thiadiazoles, à la condition que $R_3$ Soit autre que le groupe (imidazolyl-4)-méthyle.
- $R_4$ représente un hydroxyle, un alcoxy inférieur, un benzyloxy ou un groupe amino libre ou substitué par 1 ou 2 alkyles inférieurs.
- $Z_1$ représente l'isopropyle, le phényle ou le cyclohexyle formant respectivement avec le radical

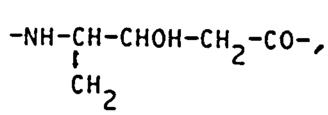

le résidu de l'aminoacide statine, à savoir, l'acide (3S, 4S) amino-4 hydroxy-3 méthyl-6 heptanoïque ou de l'acide (3S, 4S) amino-4 hydroxy-3 phényl-5 pentanoïque (AHPPA) ou de l'acide (3S, 4S) amino-4 cyclohexyl-5 hydroxy-3 pentanoïque (ACHPA) ;
- X-Y est un dipeptide choisi parmi Ala-Sta, Ala-Leu, Leu-Phe, Val-Sta, Abu-Sta, Ile-Ser, Phg-Sta ;

ainsi que les sels éventuels pharmaceutiquement acceptables dudit dérivé peptidique avec les acides minéraux ou organiques ou des métaux alcalins ou alcalino-terreux, caractérisé en ce qu'à partir d'un composé de formule : H-Y-R'$_4$, dans laquelle R'$_4$ représente un alcoxy contenant jusqu'à 6 atomes de

carbone, un benzyloxy ou un groupe amino libre ou substitué par 1 ou 2 alkyles contenant jusqu'à 6 atomes de carbone et Y est choisi parmi les résidus des acides aminés Sta, Leu, Phe et Ser, on couple, étape par étape, les divers acides aminés ou des fragments de la séquence dudit peptide convenablement protégés, le produit obtenu à chaque étape étant déprotégé, selon les procédés connus, avant d'être soumis à un nouveau couplage, chacune des opérations de couplage étant effectuée en utilisant soit un ester activé de l'aminoacide à coupler, soit l'aminoacide N-protégé en présence de dicyclohexylcarbodiimide, en ce qu'éventuellement on saponifie le composé obtenu pour former le peptide de formule I dans laquelle $R_4$ = OH et/ou en ce qu'éventuellement on transforme le peptide obtenu en l'un de ses sels pharmaceutiquement acceptables avec des acides minéraux ou organiques ou des métaux alcalins ou alcalino-terreux.

**2.** Procédé selon la revendication 1, caractérisé en ce que le dernier acide aminé à coupler est choisi de sorte que $R_1$ représente l'un des groupements suivants :

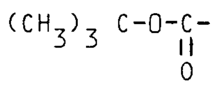

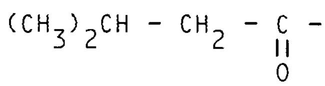

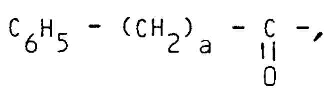 où a = 0, 1 ou 2

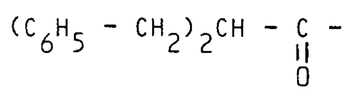

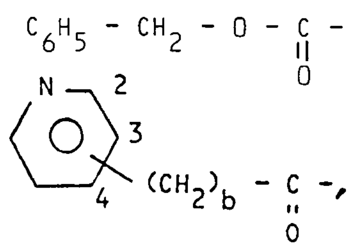 où n = 0,1,2,3,4,5 ou 6

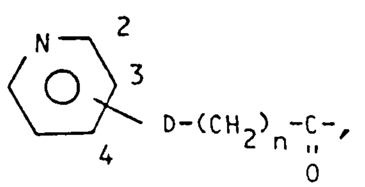 où D = 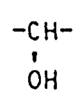 ou 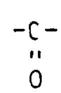

et n = 1,2,3,4 ou 5

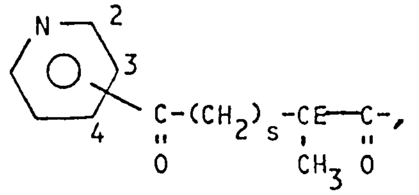 où E = -H ou $-CH_3$

et s = 1,2,3 ou 4

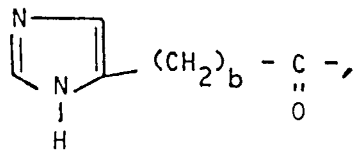 où n = 0,1,2,3,4,5 ou 6

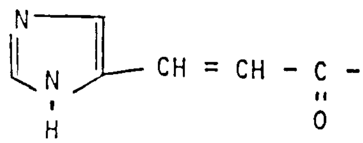

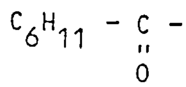

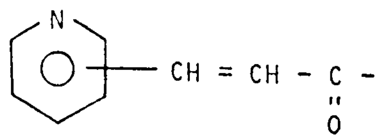

$NH_2CH_2CH_2$ - $SO_2$ -
$BocNHCH_2CH_2$ - $SO_2$ -
Z-$NHCH_2CH_2$ - $SO_2$ -
A-$SO_2$ - où A est un alkyle inférieur
$C_6H_5$ - $(CH_2)_a$ - $SO_2$, où a = 0,1 ou 2.

**3.** Procédé selon la revendication 1, caractérisé en ce que le dernier acide aminé à coupler est choisi de sorte que $R_1$ représente l'un des groupements suivants :
- isovaléryle,
- (pyridyl-2)-4 oxo-4 butyryle,
- (pyridyl-2)-4 hydroxy-4 butyryle,
- (pyridyl-3)-3 propionyle,
- (pyridyl-3)-4 butyryle,
- nicotinoyle,
- benzène sulfonyle.

**4.** Procédé selon la revendication 1, caractérisé en ce que l'un des acides aminés à coupler est choisi de sorte que le résidu -NH-CH($R_3$)-CO- représente le résidu (tauMe)His.

**Claims**
**Claims for the following Contracting States : Be, CH, DE, FR, GB, IT, LI, LU, NL, SE**

**1.** Peptide derivative :

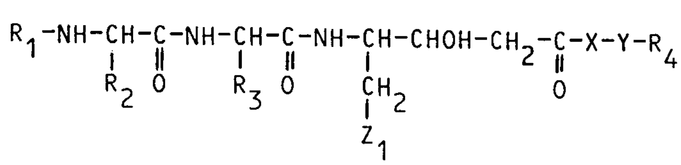 (I)

in which :
- $R_1$ represents an acyl group chosen from the following groups : alkylcarbonyl, alkoxycarbonyl, arylcarbonyl, arylalkylcarbonyl, arylalkoxycarbonyl, heterocyclylcarbonyl, heterocyclylalkylcarbonyl, in which the alkyl group designates the saturated or unsaturated aliphatic hydrocarbon radicals containing 1 à 10 carbon atoms, said alkyl group being optionally substituted by a hydroxyl, heterocyclylcarbonylalkylcarbonyl, heterocyclylalkenylcarbonyl, cycloalkylcarbonyl

group or an (alkyl containing up to 6 carbon atoms)-sulfonyl group which is unsubstituted or substituted on the alkyl by a free amino group or an amino group carrying a protecting group, or by a phenyl ; or a phenylsulfonyl group which is unsubstituted or substituted on the phenyl nucleus by an alkyl containing up to 6 carbon atoms ;

- $R_2$ represents an alkyl group containing up to 6 carbon atoms which is unsubstituted or substituted by a phenyl, naphthyl, cyclohexyl or pyridyl, or $R_2$ represents a phenyl, naphthyl, cyclohexyl or pyridyl radical ;
- $R_3$ represents an alkenyl containing up to 6 carbon atoms, a phenyl, a naphthyl, a cycloalkyl containing 3 to 6 carbon atoms, a 5-membered or 6-membered monocyclic heterocyclic group which is unsubstituted or substituted by an alkyl containing up to 6 carbon atoms or trifluoromethyl, chosen from the group comprising pyrrolidine, imidazol, thiazol, thiophene, furan, pyrrol, triazol, oxazol, isoxazol, pyridine, thiadazols ; or alternatively an alkyl containing up to 6 carbon atoms substituted by a di(alkyl containing up to 6 carbon atoms)-amino group, by a carboxyl esterified by an alkyl containing up to 6 carbon atoms or a benzyl, by a free carbamoyl or a carbamoyl substituted by one or two alkyls containing up to 6 carbon atoms or by a phenyl, by an alkoxy group containing up to 6 carbon atoms or a benzyloxy group, by a pyridylmethoxy group, by an (alkyl containing up to 6 carbon atoms)-sulfinyl or (alkyl containing up to 6 carbon atoms)-sulfonyl group, by a cycloalkyl containing from 3 to 6 carbon atoms, or by a heterocyclic group, which is unsubstituted or substituted by an alkyl containing up to 6 carbon atoms or a trifluoromethyl, chosen from the group comprising pyrrolidine, imidazol, thiazol, thiophene, furan, pyrrol, triazol, oxazol, isoxazol, pyridine, thiadazols, provided that $R_3$ is not the (imidazol-4-yl)-méthyl group ;
- $R_4$ represents a hydroxyl, an alkoxy containing up to 6 carbon atoms, a benzyloxy or a free amino group or an amino group substituted by one or 2 alkyls containing up to 6 carbon atoms ;
- $Z_1$ represents isopropyl, phenyl or cyclohexyl, respectively forming with the radical :

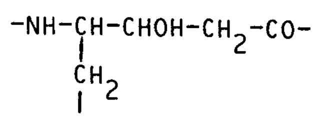

the residue of the amino acid statin, namely (3S,4S)-4-amino-3-hydroxy-6-methylheptanoic acid, of (3S,4S)-4-amino-3-hydroxy-5-phenylpentanoic acid (AHPPA) or of (3S,4S)-4-amino-5-cyclohexyl-3-hydroxypentanoic acid (ACHPA) ; and

- X-Y is a dipeptide chosen from the group comprising Ala-Sta, Ala-Leu, Leu-Phe, Val-Sta, Abu-Sta, Ile-Ser and Phg-Sta ; as well as any pharmaceutically acceptable salts of said peptide derivative with mineral or organic acids or alkali metals or alkaline earth metals.

**2.** Peptide derivative according to claim 1, characterized in that $R_1$ represents one of the following groups :

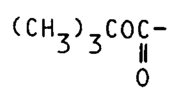

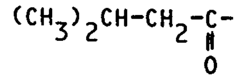

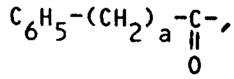 in which a = 0, 1 or 2

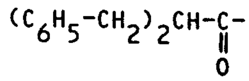

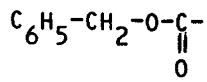

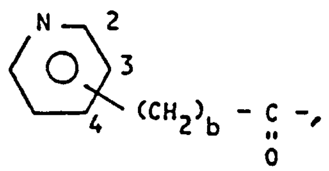 in which n = 0, 1, 2, 3, 4, 5 or 6

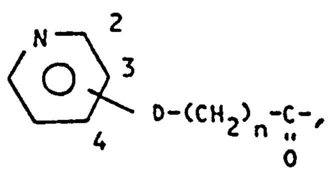 in which D = 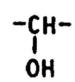 or 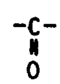

and n = 1, 2, 3, 4 or 5

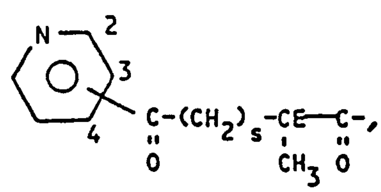 in which E = -H or $-CH_3$ and s = 1, 2, 3 or 4

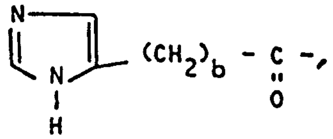 in which n = 0, 1, 2, 3, 4, 5 or 6

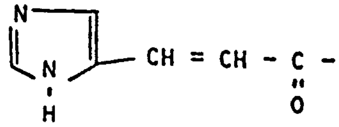

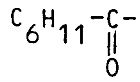

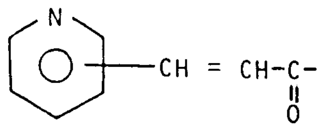

$NH_2CH_2CH_2-SO_2-$
$BocNHCH_2CH_2-SO_2-$
$ZNHCH_2CH_2-SO_2-$
$ASO_2-$, in which A is a lower alkyl
$C_6H_5(CH_2)-SO_2-$, in which a = 0, 1 or 2,
or one of any pharmaceutically acceptable salts of said peptide derivative with mineral or organic acids or with alkali metals or alkaline earth metals.

**3.** Peptide derivative according to claim 1, wherein $R_1$ is chosen from the following groups :
- isovaleryl,
- 4-(pyridin-2-yl)-4-oxobutyryl,
- 4-(pyridin-2-yl)-4-hydroxybutyryl,
- 3-(pyridin-3-yl)propionyl,
- 4-(pyridin-3-yl)butyryl,
- nicotinoyl,
- benzenesulfonyl.

**4.** Peptide derivative according to claim 1, characterized in that the residue $-NH-CH(R_3)-CO-$ represents the residue (tauMe)-His.

**5.** Process for the preparation of a peptide derivative according to claim 1, characterized in that starting from a compound of the formula :

$H-Y-R'_4$

in which $R'_4$ is an alkoxy containing up to 6 carbon atoms, a benzyloxy or a free amino group or an amino group substituted by one or two alkyls containing up to 6 carbon atoms, and Y is chosen from the residues of the amino acids Sta, Leu, Phe and Ser, the various amino acids or fragments of the sequence of the said peptide, appropriately protected, are coupled in stepwise fashion, the product obtained at each step being de-protected by known processes before being subjected to further coupling, each of the coupling operations being carried out using either an activated ester of the amino acid to be coupled, or the N-protected amino acid in the presence of dicyclohexylcarbodiimide, in that, if appropriate, the compound obtained is saponified to form the peptide of formula (I) in which $R_4$ = OH, and/or in that, if appropriate, the peptide obtained is converted to one of its pharmaceutically acceptable salts with mineral or organic acids or alkali metals or alkaline earth metals.

**6.** Pharmaceutical compositions which contains, as the active ingredient, a product according to claim 1.

**7.** Pharmaceutical compositions according to claim 6, which can be used especially for the treatment of arterial tension, characterized in that they contain from 1 to 1 000 mg of a product according to claim 1, per dosage unit, mixed with a pharmaceutical excipient.

**Claims for the following Contracting State : AT**

**1.** Process for obtaining peptide derivatives corresponding to the formula :

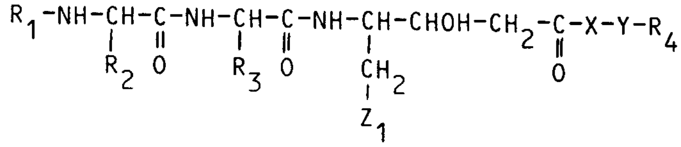 (I)

in which :

- $R_1$ represents an acyl group chosen from the following groups : alkylcarbonyl, alkoxycarbonyl, arylcarbonyl, arylalkylcarbonyl, arylalkoxycarbonyl, heterocyclylcarbonyl, heterocyclylalkylcarbonyl, in which the alkyl group designates the saturated or unsaturated aliphatic hydrocarbon radicals containing 1 à 10 carbon atoms, said alkyl group being optionally substituted by a hydroxyl, heterocyclylcarbonylalkylcarbonyl, heterocyclylalkenylcarbonyl, cycloalkylcarbonyl group or an (alkyl containing up to 6 carbon atoms)-sulfonyl group which is unsubstituted or substituted on the alkyl by a free amino group or an amino group carrying a protecting group, or by a phenyl ; or a phenylsulfonyl group which is unsubstituted or substituted on the phenyl nucleus by an alkyl containing up to 6 carbon atoms ;
- $R_2$ represents an alkyl group containing up to 6 carbon atoms which is unsubstituted or substituted by a phenyl, naphthyl, cyclohexyl or pyridyl, or $R_2$ represents a phenyl, naphthyl, cyclohexyl or pyridyl radical ;
- $R_3$ represents an alkenyl containing up to 6 carbon atoms, a phenyl, a naphthyl, a cycloalkyl containing 3 to 6 carbon atoms, a 5-membered or 6-membered monocyclic heterocyclic group which is unsubstituted or substituted by an alkyl containing up to 6 carbon atoms or trifluoromethyl, chosen from the group comprising pyrrolidine, imidazol, thiazol, thiophene, furan, pyrrol, triazol, oxazol, isoxazol, pyridine, thiadazols ; or alternatively an alkyl containing up to 6 carbon atoms substituted by a di(alkyl containing up to 6 carbon atoms)-amino group, by a carboxyl esterified by an alkyl containing up to 6 carbon atoms or a benzyl, by a free carbamoyl or a carbamoyl substituted by one or two alkyls containing up to 6 carbon atoms or by a phenyl, by an alkoxy group containing up to 6 carbon atoms or a benzyloxy group, by a pyridylmethoxy group, by an (alkyl containing up to 6 carbon atoms)-sulfinyl or (alkyl containing up to 6 carbon atoms)-sulfonyl group, by a cycloalkyl containing from 3 to 6 carbon atoms, or by a 5-membered or 6-membered monocyclic heterocyclic group, which is unsubstituted or substituted by an alkyl containing up to 6 carbon atoms or a trifluoromethyl, chosen from the group comprising pyrrolidine, imidazol, thiazol, thiophene, furan, pyrrol, triazol, oxazol, isoxazol, pyridine, thiadazols, provided that $R_3$ is not the (imidazol-4-yl)-methyl group ;
- $R_4$ represents a hydroxyl, a lower alkoxy, a benzyloxy or a free amino group or an amino group substituted by one or 2 lower alkyls ;
- $Z_1$ represents isopropyl, phenyl or cyclohexyl, respectively forming with the radical :

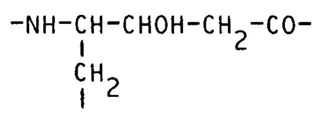

the residue of the amino acid statin, namely (3S,4S)-4-amino-3-hydroxy-6-methylheptanoic acid, of (3S,4S)-4-amino-3-hydroxy-5-phenylpentanoic acid (AHPPA) or of (3S,4S)-4-amino-5-cyclohexyl-3-hydroxypentanoic acid (ACHPA) ;

- X-Y is a dipeptide chosen from the group comprising Ala-Sta, Ala-Leu, Leu-Phe, Val-Sta, Abu-Sta, Ile-Ser and Phg-Sta ;

as well as any possibly pharmaceutically acceptable salts of said peptide derivative with mineral or organic acids or alkali metals or alkaline earth metals, characterized in that starting from a compound of the formula :

$H\text{-}Y\text{-}R'_4$

in which $R'_4$ is an alkoxy containing up to 6 carbon atoms, a benzyloxy or a free amino group or an

amino group substituted by one or two alkyls containing up to 6 carbon atoms, and Y is chosen from the residues of the amino acids Sta, Leu, Phe and Ser, the various amino acids or fragments of the sequence of the said peptide, appropriately protected, are coupled in stepwise fashion, the product obtained at each step being de-protected by known processes before being subjected to further coupling, each of the coupling operations being carried out using either an activated ester of the amino acid to be coupled, or the N-protected amino acid in the presence of dicyclohexylcarbodiimide, in that, if appropriate, the compound obtained is saponified to form the peptide of formula (I) in which $R_4$ = OH, and/or in that, if appropriate, the peptide obtained is converted to one of its pharmaceutically acceptable salts with mineral or organic acids or alkali metals or alkaline earth metals.

**2.** Process according to claim 1, characterized in that the last amino acid to be coupled is chosen in such a way that $R_1$ represents one of the following groups :

$$(CH_3)_3CO\underset{\underset{O}{\|}}{C}-$$

$$(CH_3)_2CH-CH_2-\underset{\underset{O}{\|}}{C}-$$

$$C_6H_5-(CH_2)_a-\underset{\underset{O}{\|}}{C}-,$$ in which a = 0, 1 or 2

$$(C_6H_5-CH_2)_2CH-\underset{\underset{O}{\|}}{C}-$$

$$C_6H_5-CH_2-O-\underset{\underset{O}{\|}}{C}-$$

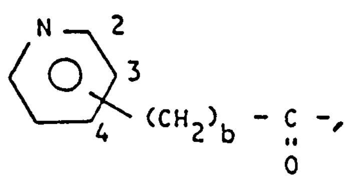 in which n = 0, 1, 2, 3, 4, 5 or 6

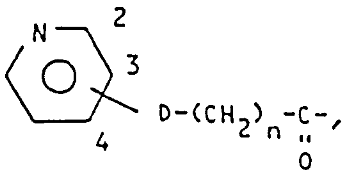 in which D = 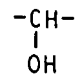 or 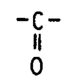

and n = 1, 2, 3, 4 or 5

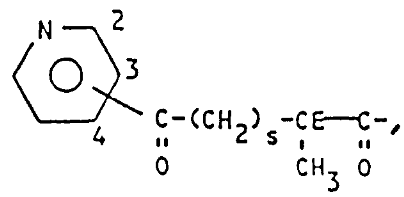 in which E = -H or $-CH_3$ and s = 1, 2, 3 or 4

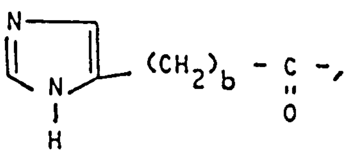 in which n = 0, 1, 2, 3, 4, 5 or 6

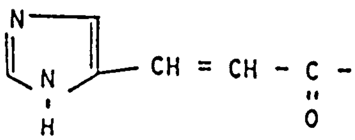

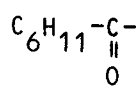

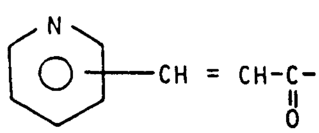

$NH_2CH_2CH_2-SO_2-$
$BocNHCH_2CH_2-SO_2-$
$ZNHCH_2CH_2-SO_2-$
$ASO_2-$, in which A is a lower alkyl
$C_6H_5-(CN_2)_a-SO_2-$, in which a = 0, 1 or 2,

**3.** Process according to claim 1, characterized in that the last amino acid to be coupled is chosen in such a way that $R_1$ is one of the following groups :
- isovaleryl,
- 4-(pyridin-2-yl)-4-oxobutyryl,
- 4-(pyridin-2-yl)-4-hydroxybutyryl,
- 3-(pyridin-3-yl)propionyl,
- 4-(pyridin-3-yl)butyryl,
- nicotinoyl,
- benzenesulfonyl.

**4.** Process according to claim 1, characterized in that one of the amino acids to be coupled is chosen in such a way that the residue $-NH-CH(R_3)-CO-$ represents the residue (tauMe)His.

**Patentansprüche**
**Patentansprüche für folgende Vertragsstaaten : BE, CH, DE, FR, GB, IT, LI, LU, NL, SE**

1. Peptidderivat

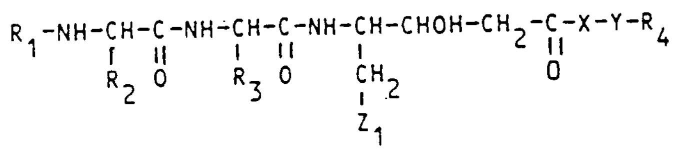

(1)

worin

$R_1$ eine Acylgruppe darstellt, ausgewählt aus den Gruppen Alxylcarbonyl, Alkoxycarbonyl, Arylcarbonyl, Arylalkylcarbonyl, Arylalkoxycarbonyl, Heterocyclylcarbonyl, Heterocyclylalkylcarbonyl, in der die Alkylgruppe gesättigte oder ungesättigte aliphatische Kohlenwasserstoffgruppen mit 1 bis 10 Kohlenstoffatomen bezeichnet und die Alkylgruppe gegebenenfalls durch eine Gruppe Hydroxy, Heterocyclylcarbonylalkylcarbonyl, Heterocyclylalkenylcarbonyl, Cycloalkylcarbonyl oder nicht substituierte oder am Alkyl durch eine freie oder eine Schutzgruppe tragende Aminogruppe oder eine durch ein Phenyl substituierte (Alkyl mit bis zu 6 Kohlenstoffatomen)-Sulfonylgruppe substituiert ist; oder eine nicht substituierte oder am Phenylkern durch ein Alkyl mit bis zu 6 Kohlenstoffatomen substituierte Phenylsulfonylgruppe;

$R_2$ eine Alkylgruppe mit bis zu 6 Kohlenstoffatomen, nicht substituiert oder durch ein Phenyl, Naphthyl, Cyclohexyl oder Pyridyl substituiert, darstellt, oder $R_2$ einen Phenyl-, Naphthyl-, Cyclohexyl- oder Pyridylrest darstellt;

$R_3$ ein Alkenyl mit bis zu 6 Kohlenstoffatomen, Phenyl, Naphthyl, Cycloalkyl mit 3 bis 6 Kohlenstoffatomen, eine nicht substituierte oder mit einem Alkyl mit bis zu 6 Kohlenstoffatomen oder Trifluormethyl substituierte monocyclische heterocyclische Gruppe mit 5 or 6 Ketten, ausgewählt aus Pyrrolidin, Imidazol, Thiazol, Thiophen, Furan, Pyrrol, Triazol, Oxazol, Isoxazol, Pyridin, Thiadiazolen; oder aber ein Alkyl mit bis zu 6 Kohlenstoffatomen, substituiert durch eine Di-(Alkyl mit bis zu 6 Kohlenstoffatomen )-aminogruppe, durch ein mit einem Alkyl bis zu 6 Kohlenstoffatomen oder ein Benzyl verestertes Carboxyl, durch ein freies oder durch ein oder zwei Alkyle mit bis zu 6 Kohlenstoffatomen oder ein Phenyl substituiertes Carbamoyl, durch eine Alkoxygruppe mit bis zu 6 Kohlenstoffatomen oder Benzyloxy, durch eine Pyridylmethyloxygruppe, durch eine (Alkyl mit bis zu 6 Kohlenstoffatomen)-Sulfinyl- oder (Alkyl mit bis zu 6 Kohlenstoffatomen)-Sulfonylgruppe, durch ein Cycloalkyl mit 3 bis 6 Kohlenstoffatomen oder durch eine nicht substituierte oder mit einem Alkyl mit bis zu 6 Kohlenstoffatomen oder Trifluormethyl substituierte heterocyclische Gruppe, ausgewählt aus Pyrrolidin, Imidazol, Thiazol, Thiophen, Furan, Pyrrol, Triazol, Oxazol, Isoxazol, Pyridin, Thiadiazolen, darstellt, mit der Maßgabe, daß $R_3$ nicht die Gruppe (4-Imidazolyl)-methyl ist;

$R_4$ Hydroxyl, Alkoxy mit bis zu 6 Kohlenstoffatomen, Benzyloxy oder eine freie oder durch 1 oder 2 Alkyle mit bis zu 6 Kohlenstoffatomen substituierte Aminogruppe darstellt;

$Z_1$ Isopropyl, Phenyl oder Cyclohexyl darstellt und mit der Gruppe

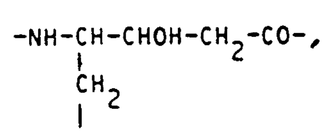

den Rest der Aminosäure Statin, nämlich (3S,4S)-4-Amino-3-hydroxy-6-methyl-heptansäure oder (3S,4S)-4-Amino-3-hydroxy-5-phenyl-pentansäure (AHPPA) oder (3S,4S)-4-Amino-5-cyclohexyl-3-hydroxy-pentansäure (ACHPA) bildet;

X-Y ein Dipeptid ist, ausgewählt aus Ala-Sta, Ala-Leu, Leu-Phe, Val-Sta, Abu-Sta, Ile-Ser, Phg-Sta;
sowie die möglichen pharmazeutisch akzeptablen Salze dieses Peptidderivats mit Mineral- oder organischen Säuren oder Alkali- oder Erdalkalimetallen.

2. Peptidderivat nach Anspruch 1, dadurch gekennzeichnet, daß $R_1$ eine der folgenden Gruppen darstellt:

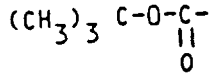

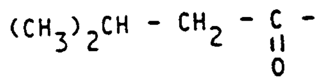

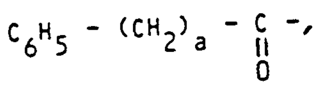 , worin a = 0,1 oder 2

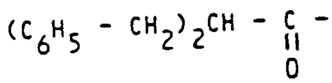

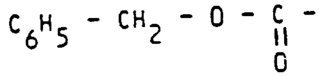

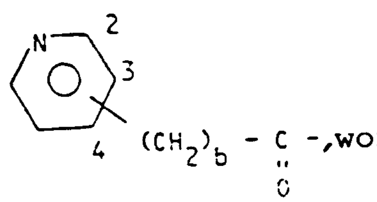 , worin n = 0,1,2,3,4,5 oder 6

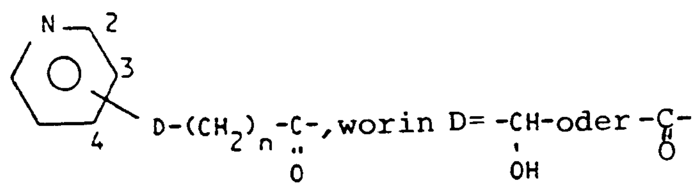 , worin D= oder

und n = 1,2,3,4 oder 5

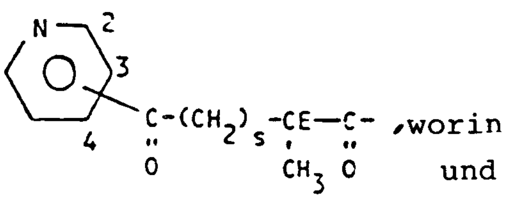 , worin E = -H oder $-CH_3$ und s = 1,2,3 oder 4

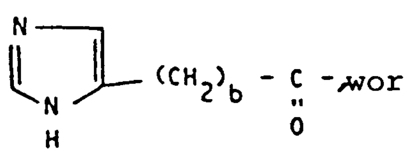 , worin n= 0,1,2,3,4,5 oder 6

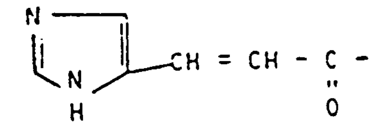

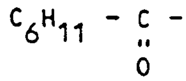

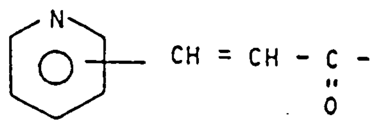

$NH_2CH_2CH_2$ - $SO_2$ -
$BocNHCH_2CH_2$ - $SO_2$ -
Z-$NHCH_2CH_2$ - $SO_2$ -
A-$SO_2$ - worin A Niedrigalkyl ist
$C_6H_5$ - $(CH_2)_a$ - $SO_2$,worin a = 0,1 oder 2
oder eines der möglichen pharmazeutisch akzeptablen Salze des Peptidderivats mit Mineral- oder organischen Säuren oder mit Alkali- oder Erdalkalimetallen.

**3.** Peptidderivat nach Anspruch 1, dadurch gekennzeichnet, daß $R_1$ ausgewählt ist aus den folgenden Gruppen:
- Isovaleryl
- 4-(2-Pyridyl)-4-oxo-butyryl
- 4-(2-Pyridyl)-4-hydroxy-butyryl
- 3-(3-Pyridyl)-propionyl
- 4-(3-Pyridyl)-butyryl
- Nicotinoyl
- Benzolsulfonyl.

**4.** Peptidderivat nach Anspruch 1, dadurch gekennzeichnet, daß der Rest -NH-CH($R_3$)-CO- den Rest (tauMe)His darstellt.

**5.** Verfahren zur Herstellung eines Peptidderivats nach Anspruch 1, dadurch gekennzeichnet, daß man, ausgehend von einer Verbindung der Formel H-Y-R'$_4$, worin R'$_4$ ein Alkoxy mit bis zu 6 Kohlenstoffatomen, ein Benzyloxy oder eine freie oder durch 1 oder 2 Alkyle mit bis zu 6 Kohlenstoffatomen substituierte Aminogruppe darstellt und Y ausgewählt ist aus den Resten der Aminosäuren Sta, Leu, Phe und Ser, stufenweise die verschiedenen Aminosäuren oder Fragmente der Sequenz des Peptids, die zweckmäßig geschützt sind, koppelt, wobei das in jeder Stufe erhaltene Produkt gemäß bekannter Verfahren entschützt wird, bevor es einer weiteren Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird, daß man gegebenenfalls die erhaltene Verbindung zur Bildung des Peptids der Formel I, in der $R_4$ = OH verseift, und/oder daß man gegebenenfalls das erhaltene Peptid mit Mineral- oder organischen Säuren oder Alkali- oder Erdalkalimetallen in eines seiner pharmazeutisch akzeptablen Salze überführt.

**6.** Pharmazeutische Zusammensetzungen, dadurch gekennzeichnet, daß sie als aktives Ingrediens ein Produkt nach Anspruch 1 enthalten.

**7.** Pharmazeutische Zusammensetzungen nach Anspruch 6, die insbesondere zur Behandlung des arteriellen Drucks geeignet sind, dadurch gekennzeichnet, daß sie 1 bis 1000 mg des Produkts nach Anspruch 1 pro Dosiseinheit in Mischung mit einem pharmazeutischen Exzipienten enthalten.

**Patentansprüche für folgenden Vertragsstaat : AT**

**1.** Verfahren zur Herstellung von Peptidderivaten der Formel

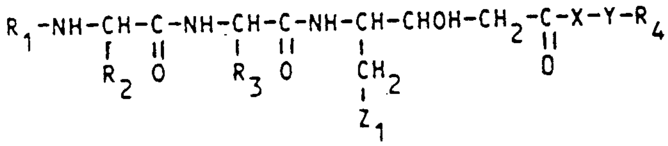 (1)

worin

R₁ eine Acylgruppe darstellt, ausgewählt aus den Gruppen Alkylcarbonyl, Alxoxycarbonyl, Arylcarbonyl, Arylalkylcarbonyl, Arylalkoxycarbonyl, Heterocyclylcarbonyl, Heterocyclylalkylcarbonyl, in der die Alkylgruppe gesättigte oder ungesättigte aliphatische Kohlenwasserstoffgruppen mit 1 bis 10 Kohlenstoffatomen bezeichnet und die Alkylgruppe gegebenenfalls durch eine Gruppe Hydroxy, Heterocyclylcarbonylalkylcarbonyl, Heterocyclylalkenylcarbonyl, Cycloalkylcarbonyl oder nicht substituierte oder am Alkyl durch eine freie oder eine Schutzgruppe tragende Aminogruppe oder eine durch ein Phenyl substituierte (Alkyl mit bis zu 6 Kohlenstoffatomen)-Sulfonylgruppe substituiert ist; oder eine nicht substituierte oder am Phenylkern durch ein Alkyl mit bis zu 6 Kohlenstoffatomen substituierte Phenylsulfonylgruppe;

$R_2$ eine Alkylgruppe mit bis zu 6 Kohlenstoffatomen, nicht substituiert oder durch ein Phenyl, Naphthyl, Cyclohexyl oder Pyridyl substituiert, darstellt, oder $R_2$ einen Phenyl-, Naphthyl-, Cyclohexyl- oder Pyridylrest darstellt;

$R_3$ ein Alkenyl mit bis zu 6 Kohlenstoffatomen, Phenyl, Naphthyl, Cycloalkyl mit 3 bis 6 Kohlenstoffatomen, eine monocyclische heterocyclische Gruppe mit 5 oder 6 Ketten, nicht substituiert oder durch ein Alkyl mit bis zu 6 Kohlenstoffatomen oder Trifluormethyl substituiert, ausgewählt aus Pyrrolidin, Imidazol, Thiazol, Thiophen, Furan, Pyrrol, Triazol, Oxazol, Isoxazol, Pyridin, Thiadiazolen; oder aber ein Alkyl mit bis zu 6 Kohlenstoffatomen, substituiert durch eine Di-(Alkyl mit bis zu 6 Kohlenstoffatomen )-aminogruppe, durch ein mit einem Alkyl bis zu 6 Kohlenstoffatomen oder ein Benzyl verestertes Carboxyl, durch ein freies oder durch ein oder zwei Alkyle mit bis zu 6 Kohlenstoffatomen oder ein Phenyl substituiertes Carbamoyl, durch eine Alkoxygruppe mit bis zu 6 Kohlenstoffatomen oder Benzyloxy, durch eine Pyridylmethyloxygruppe, durch eine (Alkyl mit bis zu 6 Kohlenstoffatomen)-Sulfinyl- oder (Alkyl mit bis zu 6 Kohlenstoffatomen)-Sulfonylgruppe, durch ein Cycloalkyl mit 3 bis 6 Kohlenstoffatomen oder durch eine nicht substituierte oder mit einem Alkyl mit bis zu 6 Kohlenstoffatomen oder Trifluormethyl substituierte monozyklische heterozyklische Gruppe mit 5 oder 6 Ketten darstellt, ausgewält aus Pyrrolidin, Imidazol, Thiazol, Thiophen, Furan, Pyrrol, Triazol, Oxazol, Isoxazol, Pyridin, Thiadiazolen ; mit der Maßgabe, daß $R_3$ nicht die Gruppe (4-Imidazolyl)-methyl ist,

$R_4$ Hydroxyl, Niedrigalkoxy, Benzyloxy oder eine freie oder durch 1 oder 2 Niedrigalkyle substituierte Aminogruppe darstellt;

$Z_1$ Isopropyl, Phenyl oder Cyclohexyl darstellt und mit der Gruppe

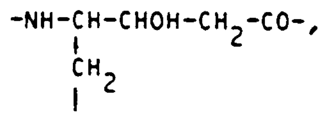

den Rest der Aminosäure Statin, nämlich (3S,4S)-4-Amino-3-hydroxy-6-methyl-heptansäure oder (3S,4S)-4-Amino-3-hydroxy-5-phenyl-pentansäure (AHPPA) oder (3S,4S)-4-Amino-5-cyclohexyl-3-hydroxy-pentansäure (ACHPA) bildet;

X-Y ein Dipeptid ist, ausgewählt aus Ala-Sta, Ala-Leu, Leu-Phe, Val-Sta, Abu-Sta, Ile-Ser, Phg-Sta;

sowie der möglichen pharmazeutisch akzeptablen Salze dieses Peptidderivats mit Mineral- oder organischen Säuren oder Alkali- oder Erdalkalimetallen, dadurch gekennzeichnet, daß man, ausgehend von einer Verbindung der Formel H-Y-R'₄, worin R'₄ ein Alkoxy mit bis zu 6 Kohlenstoffatomen, ein Benzyloxy oder eine freie oder durch 1 oder 2 Alkyle mit bis zu 6 Kohlenstoffatomen substituierte Aminogruppe darstellt und Y ausgewählt ist aus den Resten der Aminosäuren Sta, Leu, Phe und Ser,

stufenweise die verschiedenen Aminosäuren oder Fragmente der Sequenz des Peptids, die zweckmäßig geschützt sind, koppelt, wobei das in jeder Stufe erhaltene Produkt gemäß bekannter Verfahren entschützt wird, bevor es einer weiteren Kopplung unterzogen wird, wobei jeder Kopplungsvorgang unter Verwendung entweder eines aktivierten Esters der zu koppelnden Aminosäure oder der N-geschützten Aminosäure in Gegenwart von Dicyclohexylcarbodiimid durchgeführt wird, daß man gegebenenfalls die erhaltene Verbindung zur Bildung des Peptids der Formel I, in der $R_4$ = OH verseift, und/oder daß man gegebenenfalls das erhaltene Peptid mit Mineral-oder organischen Säuren oder Alkali- oder Erdalkalimetallen in eines seiner pharmazeutisch akzeptablen Salze überführt.

**2.** Verfahren nach Anspruch 1, dadurch gekennzeichnet, daß die letzte zu koppelnde Aminosäure derart gewählt ist, daß $R_1$ eine der folgenden Gruppen darstellt:

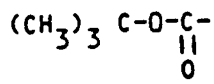

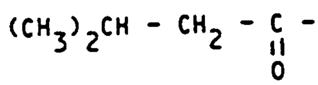

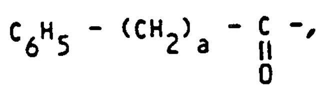 worin a = 0,1 oder 2

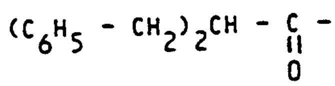

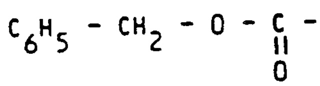

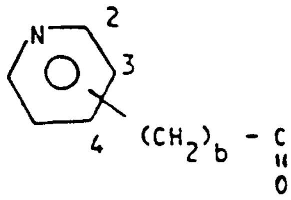

, worin n = 0,1,2,3,4,5 oder 6

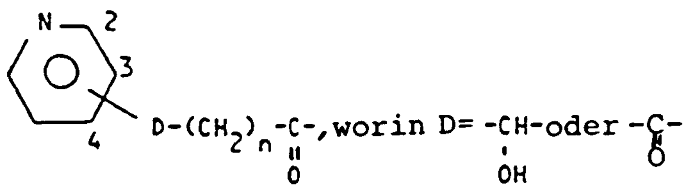

und n = 1,2,3,4 oder 5

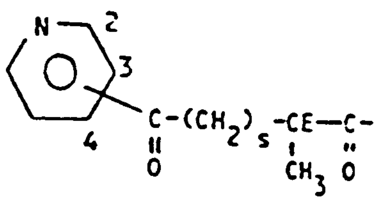

, worin E = -H oder $-CH_3$ und s = 1,2,3 oder 4

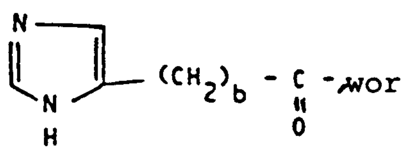

, worin n= 0,1,2,3,4,5 oder 6

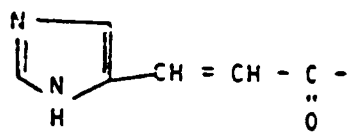

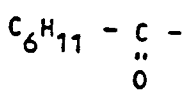

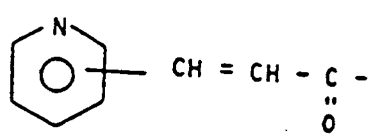

$NH_2CH_2CH_2$ - $SO_2$ -
$BocNHCH_2CH_2$ - $SO_2$ -
Z-$NHCH_2CH_2$ - $SO_2$ -
A-$SO_2$ - worin A Niedrigalky ist
$C_6H_5$ - $(CH_2)_a$ - $SO_2$,worin a = 0,1 oder 2,

**3.** Verfahren nach Anspruch 1, dadurch gekennzeichnet, daß die letzte zu koppelnde Aminosäure derart gewählt ist, daß $R_1$ eine der folgenden Gruppen darstellt:
- Isovaleryl
- 4-(2-Pyridyl)-4-oxo-butyryl
- 4-(2-Pyridyl)-4-hydroxy-butyryl
- 3-(3-Pyridyl)-propionyl
- 4-(3-Pyridyl)-butyryl
- Nicotinoyl
- Benzolsulfonyl.

**4.** Verfahren nach Anspruch 1, dadurch gekennzeichnet, daß eine der zu koppelnden Aminosäuren derart

gewählt ist, daß der Rest -NH-CH($R_3$)-CO- den Rest (tauMe)His darstellt.